# EUROPEAN PATENT APPLICATION

(11) **EP 4 026 548 A1**
(43) Date of publication of application: **13.07.2022**
(21) Application number: 20860261.5
(22) Date of filing: 03.09.2020
(51) Int. Cl.: A61K 31/519, A61P 9/00

(54) **USE OF 5-METHYLTETRAHYDROFOLATE**

(30) Priority: 03.09.2019 CN 201910828557
(71) Applicant: Lianyungang Jinkang Hexin Pharmaceutical Co. Ltd., Lianyungang, Jiangsu 222000 (CN)
(72) Inventor: CHENG, Yongzhi, Lianyungang, Jiangsu 222000 (CN); LIAN, Zenglin, Lianyungang, Jiangsu 222000 (CN); GU, Rui, Lianyungang, Jiangsu 222000 (CN)
(74) Representative: Cohausz & Florack
(86) International application number: PCT/CN2020/113157
(87) International publication number: WO 2021/043196

(57) **Abstract**

The present disclosure discloses use of 5-methyltetrahydrofolate in preparing a medicine or health-care food for preventing neonatal congenital heart disease in peri-pregnancy and/or pregnant women. The 5-methyltetrahydrofolate has an effect of preventing congenital heart disease. The prophylactic dosage of the 5-methyltetrahydrofolate can be greater than 1 mg. Compared with synthetic folic acid, high-dose use has no teratogenic effect. Thus the 5-methyltetrahydrofolate can prevent birth defects in a high dose and can be used for preventing neonatal congenital heart disease.

## Description

### TECHNICAL FIELD

The present disclosure belongs to the field of medicines and relates to new use of a composition containing 5-methyltetrahydrofolate in preparing a medicine or health-care food for preventing neonatal congenital heart disease, miscarriage and stillbirth in peri-pregnancy and/or pregnant women.

### BACKGROUND

Congenital heart disease (CHD) has become a main disease of birth defects and is the most common heart disease in childhood. CHD seriously jeopardizes the life and the life quality of child patients, and is an important reason for death of children aged 0-5 years.

The CHD is divided into various subtypes with 7 most common subtypes: ventricular septal defects (VSDs), atrial septal defects (ASDs), patent ductus arteriosus (PDA), tetralogy of Fallot (TOF), transposition of the great arteries (TGA), tricuspid valve occlusion or stenosis, and pulmonary stenosis (PS).

At present, an effect of folic acid in preventing neural tube defects has been confirmed. But experts in each country have disputed on folic acid in preventing CHD. Most scholars believe that folic acid supplementation can help prevent CHD. Some people even proposed that a higher dose of folic acid should be used to prevent CHD [Huhta JC, Linask K. When should we prescribe high-dose folic acid to prevent congenital heart defects?[J]. Current Opinion in Cardiology, 2015, 30(1):125-131]. Folic acid is pteroylglutamic acid, in a synthetic form and also called vitamin B9 and vitamin M. In medical application, folic acid is used to prevent neural tube defects of newborn and prevent and treat megaloblastic anemia. A daily intake of 400 µg/d of the folic acid for lactating mothers and pregnant women is recommended by the world health organization. High intake of the folic acid results in presence of unmetabolized folic acid and reduced folic acid (5-methyltetrahydrofolate and derivatives thereof) in blood circulation.

Folic acid is a synthetic oxidized form not found in natural food and is required to be converted to tetrahydrofolic acid by a human dihydrofolate reductase (DHFR) to be used by humans, while the process is slow in humans. Studies showed that a metabolic rate of folic acid by human liver was 1.79% of that by rats at a physiological pH. Therefore, unmetabolized folic acid may be detected in blood after folic acid administration and it was reported that the unmetabolized folic acid can be detected in plasma several hours after folic acid administration exceeding 200 µg [Bailey SW, Ayling JE. The extremely slow and variable activity of dihydrofolate reductase in human liver and its implications for high folic acid intake. Proc Natl Acad Sci U S A. 2009; 106(36):15424-15429]. In addition, accumulation of the unmetabolized folic acid in cells may lead to decreased DHFR activity.

Reviewing existing studies of association of folic acid with CHD, several studies in the early north American regions are worth mentioning. In one study, 207 mothers giving birth to infants with conotruncal heart defects and 481 randomly selected mothers giving birth to infants free of malformations in California from 1987 to 1998 were interviewed by a phone. The study showed that the infants of the mothers taking multivitamin or folic acid fortified cereals had a reduced risk of conotruncal heart defects [Shaw GM, O'Malley CD, Wasserman CR, Tolarova MM and Lammer EJ. Maternal periconeptional use of multivitamins and reduced risk for conotruncal heart defects and limb deficiencies among offspring. American Journal of Medical Genetics. 1995; 59:536-545]. But relevant estimated value of the study was not significant. Later in the study of California from 1999 to 2004, no folic acid vitamin complex was found to have a preventive effect on conotruncal heart defects [Shaw GM, Carmichael SL, Yang W and Lammer EJ. Periconceptional nutrient intakes and risks of conotruncal heart defects. Birth Defects Res A Clin Mol Teratol. 2010; 88:144=51]. A control study conducted in Boston, Philadelphia and Toronto from 1993 to 1996 showed that no significant association was found between prenatal use of folic acid containing vitamins and types of heart defects [Werler MM, Hayes C, Louik C, Shapiro S and Mitchell AA. Multivitamin supplementaion and risk of birth defects. American Journal of Epidemiology. 1999; 150:675-682]. In a study of Baltimore-Washington, no prophylactic effect of 400 mg/day of folic acid supplementation before pregnancy was found [Scanlon K S, Ferencz C, Loffredo C A, et al. Preconceptional folate intake and malformations of the cardiac outflow tract. Baltimore-Washington Infant Study Group. [J]. Epidemiology, 1998, 9(1):95-98]. In a US birth defects prevention research report from 1997 to 2004, it was noted that in a multicenter study of a combined effect of maternal diabetes mellitus and various folic acid-containing vitamins on various types of heart defects, an unadjusted OR value was 0.95 (95%CI 0.85-1.06), indicating no significant association [Correa A, Gilboa SM, Botto LD, Moore CA, Hobbs CA, Cleves MA, Riehle-Colarusso TJ, Waller DK, Reece EA and National Birth Defects Prevention S. Lack of periconceptional vitamins or supplements that contain folic acid and diabetes mellitus-associated birth defects. Am J Obstet Gynecol. 2012; 206:218 e1-13].

In a random control study of folic acid supplementation for preventing neural tube malformations from 1984 to 1991, a Hungarian research team indicated that an incidence rate of neural tube malformations of an experimental group was greatly reduced and an incidence rate of CHD was also found reduced by 40% when people in an experimental group begin to take multivitamin containing 0.8 mg of folic acid from 1 month before pregnancy until 3 months after pregnancy was confirmed [Czeizel, Andrew E. Prevention of congenital abnormalities by periconceptional multivitamin supplementation. [J]. Bmj, 1993, 306(6893):1645-1648]. However, the above study had drawbacks, included cases of CHD were too few, and there were only 10 cases of exposed heart defects and 17 cases of unexposed heart defects. A subsequent evaluation study found that pregnant women with influenza and common cold accompanied by complications were associated with a higher risk of conotruncal defects, while a high dose of folic acid can reduce a risk of the conotruncal defects [Csáky-Szunyogh, Melinda, Vereczkey A, Kósa, Zsolt, et al. Risk and protective factors in the origin of conotruncal defects of heart-a population-based case-control study.[J]. American Journal of Medical Genetics Part A, 2013, 161(10):2444-2452].

An Australian study from 1997 to 1998 showed no association between folate and a risk of heart defects in offspring [Bower C, Miller M, Payne J, Serna P. Folate intake and the primary prevention of non-neural birth defects. Aust N Z J Public Health. 2006;30(3):258-261]. In a case-control study in the north of the Netherlands, it was showed that women taking folic acid had an approximately 20% lower risk of giving birth to infants with heart defects. However, an analysis of heart disease subtypes of the report, a risk of atrioventricular septal defects was increased with folic acid supplementation with an OR value of 1.28 (95%CI 0.33-4.95) [van Beynum IM, Kapusta L, Bakker MK, den Heijer M, Blom HJ, de Walle HE. Protective effect of periconceptional folic acid supplements on the risk of congenital heart defects: a registry-based case-control study in the northern Netherlands. Eur Heart J. 2010; 31(4):464-471].

A Norwegian research team pointed out that folic acid supplementation during peri-pregnancy was not found to be associated with a reduction in the incidence of severe CHD [Leirgul E, Gildestad T, Nilsen RM, et al. Periconceptional Folic Acid Supplementation and Infant Risk of Congenital Heart Defects in Norway 1999-2009 [J]. Paediatric and Perinatal Epidemiology, 2015, 29(5):391-400]. At the same time, it was found that after 0.4 mg of folic acid supplementation, a risk of septal defect CHD increased by 20%. Furthermore, in an analysis study of 197,123 infant cases in Norway from 2000 to 2009 and Denmark from 1996 to 2003, some data may include the aforementioned studies and it was concluded that folic acid was not associated with a risk of birth defects in offspring with CHD, including severe defects, conus defects or septal defects [Øyen N, Olsen SF, Basit S, et al. Association Between Maternal Folic Acid Supplementation and Congenital Heart Defects in Offspring in Birth Cohorts From Denmark and Norway. J Am Heart Assoc. 2019; 8 (6): e011615].

Scholars in Canada also counted about 200,000 births in Alberta from 1995 to 2002 and found that before and after implementation of a folic acid fortification policy in 1998, a total incidence of CHD did not change [Bedard T, Lowry RB, Sibbald B, et al. Folic acid fortification and the birth prevalence of congenital heart defect cases in Alberta, Canada[J]. Birth Defects Research Part A: Clinical and Molecular Teratology, 2013, 97 (8):564-570].

Several studies on prevention of CHD with folic acid were also been conducted in China. Hospital case-control studies in Guangdong, Hubei, Fujian and Shanxi showed that folic acid supplementation can reduce a risk of CHD by 65% [Li X, Li S, Mu D, et al. The association between periconceptional folic acid supplementation and congenital heart defects: a case-control study in China. Prev Med. 2013; 56(6):385-389]. Chinese scholar [Baohong M Jie Q, Nan Z, et al. Maternal folic acid supplementation and dietary folate intake and congenital heart defects[J]. PLOS ONE, 2017, 12(11): e0187996-.]. In a birth cohort study in Gansu Province from 2010 to 2012, it was pointed out that folic acid reduced probability of neonatal CHD and a risk of pregnant women with low intake giving child patients with CHD increased by almost 2 times compared with a folic acid supplementation group. It should be noted that the above-mentioned study report also pointed out that the intake of folic acid greater than 221.03 µg/day was associated with an increased risk of atrioventricular septal defects with an OR value of 1.20 (95% CI 0.58-2.51). In a study in Guangdong from 2004 to 2016 and based on about 8,379 CHD cases and 6,918 control cases, it was concluded that the use of folic acid in pregnant women in a first trimester was associated with a lower risk of coronary heart disease [Qu Y, Lin S, Zhuang J, et al. First-Trimester Maternal Folic Acid Supplementation Reduced Risks of Severe and Most Congenital Heart Diseases in Offspring: A Large Case-Control Study. J Am Heart Assoc. 2020; 9(13): e015652]. An explanation for inconsistency of data with predecessors may be that prior to a free folic acid supplementation program in 2010 in China, few pregnant women used folic acid and had a low folic acid level. Besides, the folic acid level in diets of pregnant women and the folic acid level of the pregnant women themselves were not investigated in the study, and dose and other relevant factors were not discussed. Furthermore, the authors acknowledged that only a few of statistical cases reflected subtypes of the CHD and may not be representative.

A prerequisite for discussing cause and effect is to design studies with specific information on use of folic acid supplementation and a risk of heart defects in infants. But some of the above findings are contradictory due to different designs, bias or confusion and it can also be explained by differences in levels of folic acid in regional dietary habits.

In conclusion, an effect of folic acid on CHD is still questioned. In the above fifteen clinical studies related to folic acids and CHD, eight studies definitely supported that folic acids contribute to prevention of CHD, where the number of cases studied in the Hungarian study was small and research reports in the Netherlands, Norway and China showed that folic acid supplementation may increase a risk of atrioventricular septal defects although the folic acid was reported to reduce a risk of CHD. The above studies were only a clinical data statistic or a telephone follow-up study, but not a double-blind controlled trial. A further confirmation on whether the CHD is related to folic acid is required due to prejudice of the study authors on the prior art.

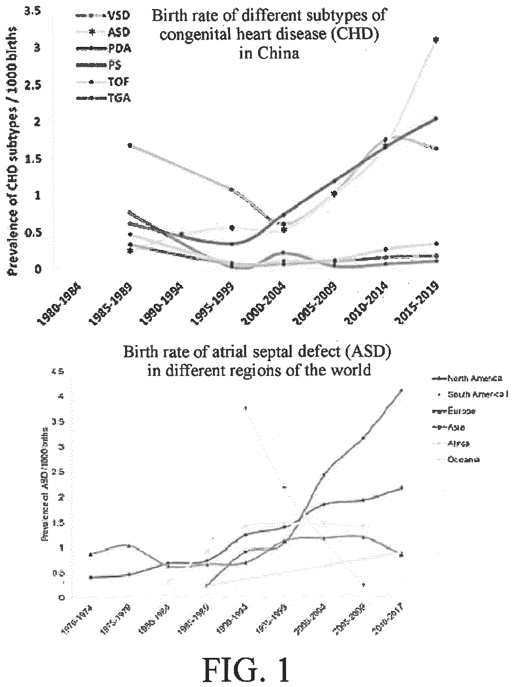

At present, the international medical community mostly recognizes that folic acid supplementation of peri-pregnancy women is helpful to reduce an occurrence risk of CHD. In China, folic acid supplementation of pregnant women is also a common consensus, but an incidence rate of the CHD is still increased. The statistics of the incidence of CHD in various countries, particularly data about atrial septal defects (ASDs) (FIG. 1) were surprising. A birth rate of the ASDs was remarkably increased in North America and Asia after 1998, while was reduced to a certain extent in Europe and South America [Liu Y, Chen S, Zühlke L, et al. Global birth prevalence of congenital heart defects 1970-2017: updated systematic review and meta-analysis of 260 studies. Int J Epidemiol. 2019; 48(2):455-463]. It is worth noting that in 1998, requirements were promulgated in the United States to enforce addition of folic acid to staple foods such as grains, flour, bread and the like and similar requirements were also introduced by Canada. There is no mandatory requirement in Europe. In addition, compliance with folic acid supplementation in European countries is very low. According to the European congenital anomalies report published in 2009, compliance with folic acid supplementation was between 5% and 40%.

In China, a birth rate related to ASDs rapidly increased after 1980. It was found in the study that an incidence rate of an urban population of CHD in China had a much higher incidence rate than that of a rural population, which had a gradually-enlarged trend. According to estimation of the world health organization, an incidence rate of birth defects is 6.42% in low-income countries, 5.57% in medium-income countries and 4.72% in high-income countries. Although China develops rapidly, and the living standard and the medical care standard are rapidly improved, particularly the developed cities in the east, such as Shanghai and South Jiangsu reaching a level of the developed countries as a whole, a birth defect rate of infants does not show a corresponding level control, but a birth defect rate of the urban population is higher than that of the rural population. Literature reports showed the incidence rate of CHD in China was greatly increased in recent years, slowly increased from 1980 to 2004, and greatly increased from 2005 to 2019. According to a study report, a birth rate of CHD in China was increased from 0.201 per thousand infants in 1980-1984 to 4.905 per thousand infants in 2015-2019 [Zhao L, Chen L, Yang T, et al. Birth prevalence of congenital heart disease in China, 1980-2019: a systematic review and meta-analysis of 617 studies. Eur J Epidemiol. 2020; 35(7):631-642]. It is worth noting that in China, from 1980 to 2019, the birth rate of ASDs increased significantly over time from about 0.25 per thousand in 1980 to 0.5 per thousand in 2000, and rose rapidly to 3 per thousand after 2000, and the ASDs almost occupied a half of the CHD.

Although China begins implementing national folic acid supplementation of pregnant woman in this period, a free folic acid policy is provided in some regions, an incidence of neural tube malformations is significantly reduced by folic acid supplementation, while an incidence of CHD is greatly increased, the inventor carefully doubts whether folic acid supplementation has a certain correlation with an increased incidence of CHD and confirms that folic acid (unmetabolized folic acid) would lead to an increased incidence of CHD in the studies disclosed by the present disclosure in combination with epidemiological statistics and local folic acid use.

Formaldehyde is a colorless irritant gas, easily dissolved in water and ethanol, and widely used in production of human industry, including chemical industry, wood industry, textile industry and anticorrosion engineering. With continuous improvement of requirements on health, people pay more and more attention to harm of formaldehyde in the environment. Formaldehyde is inevitably generated in a house decoration process and difficult to completely remove. The formaldehyde content in new houses is therefore often higher than a safe value. This also increases risks of miscarriage in pregnant women and impaired fetal health. The inventor unexpectedly discovers that 5-methyltetrahydrofolate can save zebrafish embryos affected by formaldehyde in a process of researching CHD. Meanwhile, high-concentration 5-methyltetrahydrofolate is non-toxic and free of teratogenicity.

### SUMMARY

Based on an animal experiment, it is found that if folic acid is taken during pregnancy, unmetabolized folic acid will cause teratogenicity to embryos, and long-term or excessive supplementation of folic acid may lead to occurrence of congenital heart disease. However, 5-methyltetrahydrofolate is not teratogenic and can prevent occurrence of congenital heart disease when taken during pregnancy.

The present disclosure provides a medicine or health-care food containing 5-methyltetrahydrofolate and the medicine or the health-care food is used for pregnant women to prevent neonatal congenital heart disease.

The medicine or the health-care food is used for pregnant women to prevent occurrence of neonatal congenital heart disease.

According to the medicine or the health-care food of the present disclosure, the congenital heart disease is a most common type of congenital malformations and refers to an abnormal anatomical structure caused by a formation disorder or an abnormal development of the heart and the great blood vessels during an embryonic development period or a channel that should automatically close after birth fails to close.

According to the medicine or the health-care food of the present disclosure, the congenital heart disease comprises diseases of three subgroups: 1. congenital malformation of great arteries, including patent ductus arteriosus, aortic stenosis, pulmonary artery stenosis, pulmonary atresia or other congenital malformation of great arteries; 2. congenital septal defects, including atrioventricular septal defects (AVSDs), ventricular septal defects (VSDs), atrial septal defects (ASDs), tetralogy of Fallot, aortopulmonary septal defects or other congenital septal defects; and 3. other congenital heart disease, including congenital malformations of cardiac chambers and connections, congenital aortic or mitral valve malformations.

According to the medicine or the health-care food of the present disclosure, the medicine or the health-care food contains an effective amount of 5-methyltetrahydrofolate and also contains a pharmaceutically acceptable auxiliary material or auxiliary agent.

The medicine or the health-care food of the present disclosure can be in various dosage forms known in the art. For example, enteral dosage forms, such as oral, sublingual or rectal administration; exemplarily, oral dosage forms can be a tablet, a capsule, an oral liquid, a drop pill, a pill, a powder and a granule; and exemplarily, injection forms can be a powder injection, a solution, an emulsion and a suspension.

According to the medicine or the health-care food of the present disclosure, the dosage of the 5-methyltetrahydrofolate is 0.05-50 mg/day, preferably 0.2-0.8 mg/day.

Another aspect of the present disclosure is to provide a medicine or health-care food for preventing pregnant women from miscarriage due to formaldehyde and protecting a fetus.

The medicine or the health-care food for preventing pregnant women from miscarriage due to formaldehyde and protecting a fetus can prevent the miscarriage of the pregnant women due to long-term exposure to formaldehyde.

The medicine or the health-care food for preventing pregnant women from miscarriage due to formaldehyde and protecting a fetus can be used to treat threatened miscarriage of the pregnant women caused by formaldehyde.

The medicine or the health-care food for preventing pregnant women from miscarriage due to formaldehyde and protecting a fetus can be in various dosage forms known in the art. For example, enteral dosage forms, such as oral, sublingual or rectal administration; exemplarily, oral dosage forms can be a tablet, a capsule, an oral liquid, a drop pill, a pill, a powder and a granule; and exemplarily, injection forms can be a powder injection, a solution, an emulsion and a suspension.

According to the medicine or the health-care food for preventing pregnant women from miscarriage due to formaldehyde and protecting a fetus, the dosage of the 5-methyltetrahydrofolate is 0.05-50 mg/day, preferably 0.2-0.8 mg/day, and the dosage for treating threatened miscarriage is 0.8-200 mg/day, preferably 20 mg/day.

Obviously, according to the above-mentioned content of the present disclosure and common technical knowledge and customary means in the field, without departing from the above-mentioned basic technical idea of this aspect, other modifications, substitutions and changes can also be made.

In particular, due to differences in laws and regulations of various countries, dietary supplements in the United States, for example, can claim functions (FDA agrees that in the dietary supplements, a relationship between a food or dietary component and a health status or reduction of a disease risk can be described). Therefore, the 5-methyltetrahydrofolate can be used in preparing a medicine or health-care food for preventing congenital heart disease, miscarriage and stillbirth, can also be used in preparing dietary supplements and foods for special medical purpose with the above functions, exemplarily, such as a multivitamin tablet containing 5-methyltetrahydrofolate, formula milk powder special for pregnant women and the like.

Use of 5-methyltetrahydrofolate calcium in preparing a medicine or health-care food for preventing fetal heart malformations.

The heart malformations may be preferably caused by ethanol, lead nitrate and aristolochic acid.

The 5-methyltetrahydrofolate includes 5-methyltetrahydrofolate or a pharmaceutically acceptable salt thereof.

The 5-methyltetrahydrofolate is selected from a group consisting of 5-methyl-(6S)tetrahydrofolate, 5-methyl-(6R)tetrahydrofolate, or 5-methyl(6R, S)tetrahydrofolate, i.e. including optical isomers of the 5-methyltetrahydrofolate and a mixture of the optical isomers, especially pure optical natural isomers.

The pharmaceutically acceptable salt includes a corresponding acidic salt formed by converting a basic group of the 5-methyltetrahydrofolate and a corresponding basic salt formed by converting an acidic group of the 5-methyltetrahydrofolate; and the pharmaceutically acceptable salt is preferably. a hydrochloride, a sulfate, a nitrate, a phosphate, a sodium salt, a potassium salt, a magnesium salt, a calcium salt, an ammonium salt, a substituted ammonium salt, or a salt formed with arginine or lysine.

Use of 5-methyltetrahydrofolate in preparing a medicine for preventing congenital heart disease caused by heavy metals, alcohol and medicines.

### Detailed description of the present disclosure

In the present disclosure, the term "5-methyltetrahydrofolate" includes 5-methyl-(6S)tetrahydrofolate, 5-methyl-(6R)tetrahydrofolate and 5-methyl(6R,S)tetrahydrofolate, i.e. including optical isomers of the 5-methyltetrahydrofolate, especially pure optical natural isomers, a mixture of the optical isomers, e.g. a racemic mixture, and a physiologically acceptable salt thereof, where 5-(methyl)-(6S)tetrahydrofolate is particularly preferable.

The physiologically acceptable salt refers to an acidic salt formed by converting a basic group of the 5-methyltetrahydrofolate, and the acid can be an inorganic acid, such as hydrochloric acid, sulfuric acid, nitric acid, phosphoric acid; organic acids such as formic acid, acetic acid, propionic acid, diethylacetic acid, malonic acid, succinic acid, fumaric acid, maleic acid, lactic acid, tartaric acid, malic acid, citric acid, gluconic acid, ascorbic acid or niacin, etc.

The physiologically acceptable salt can also refer to a basic salt formed by converting an acidic group of the 5-methyltetrahydrofolate, an appropriate salt, such as a sodium salt, a potassium salt, a magnesium salt, a calcium salt, an ammonium salt, a substituted ammonium salt, or a salt formed with arginine or lysine.

The folic acid is pteroylglutamic acid, in a synthetic form and also called vitamin B9 and vitamin M. The research paper showed that pregnant women taking folic acid of more than 266.5 µg daily in a first month before pregnancy had a higher incidence rate of neonatal atrioventricular septal defects (ASDs) than pregnant women taking folic acid of 115.97-265.5 µg daily. But the authors neglected the phenomenon and still thought that folic acid supplementation was beneficial to reducing an incidence rate of neonatal congenital heart disease [Baohong M, Jie Q, Nan Z, et al. Maternal folic acid supplementation and dietary folate intake and congenital heart defects[J]. PLOS ONE, 2017, 12(11): e0187996-]. 265.5 µg/day was still below the recommended intake by the United Nations, but the phenomenon was not further discussed by the authors. That folic acid supplementation will improve an incidence rate of neonatal congenital heart disease is a subversive argument and not found by people. A main reason is that an absolute value of cases with neonatal defects is very low and although a congenital heart disease birth defect is a main type, it is only less than 5‰. A clinical contrast directly used for verification needs to include tens of thousands of pregnant women, which is unrealistic. However, in a previous research report, a folic acid level and a dietary influence of the included cases cannot be controlled by a statistical analysis of medical records, such that the influence is difficult to eliminate.

Folic acid supplementation in pregnant women also results in presence of unmetabolized folic acid in blood circulation. In a study of folic acid supplementation in pregnant women in American that 0.19 nmol/L of unmetabolized folic acid was detected in serum of 25 pregnant women supplemented with folic acid during pregnancy and the folic acid had a higher concentration in umbilical cord blood of 0.27 mmol/L, suggesting that an accumulated concentration of the unmetabolized folic acid in the fetus may be greater than that in the mother [Obeid R, Kasoha M, Kirsch S H. Concentrations of unmetabolized folic acid and primary folate forms in pregnant women at delivery and in umbilical cord blood[J]. American Journal of Clinical Nutrition, 2010, 92(6):1416-1422].

The inventor discovers that folic acid has a teratogenic effect on zebrafish embryos, can cause pericardium edema of zebrafish embryos, and enables the zebrafish embryos to have bradycardia and a shortened body length. The folic acid can cause pericardium edema, pigmentation disorder and intersegmental vascular hypoplasia through microscope observation. Therefore, the folic acid can also have a teratogenic effect on human embryo development. A key period of human embryo development and heart formation is early 8 weeks. An early fetal cardiovascular circulation is formed in 6-8 weeks of pregnancy. The folic acid has a teratogenic effect on vascular development and pericardium development, and thus cause cardiovascular abnormality or diaphragm malformations of newborn can be caused, and congenital heart disease.

The inventor further observes that the 5-methyltetrahydrofolate has no teratogenic effect on the zebrafish embryos. There is no difference between a normal embryo group and a 5-methyltetrahydrofolate intervention group. The zebrafish embryo has a normal heart appearance and normal development of lower intestinal network blood vessels under an intervention of the 5-methyltetrahydrofolate.

In one example, zebrafish embryos under folic acid intervention at different times (24 h and 48 h) are collected and expressions of different transcription factors in the zebrafish embryos are detected by using a quantitative PCR techniques. As a result, the expressions of the transcription factors NKx2.5, amhc, vmhc, hand2, has2, mef2a, mef2c, bmp2b, ephrinB2 and ephB4 in the zebrafish embryos under folic acid intervention are influenced, where the NKx2.5, the hand2, the has2, the mef2c and the ephB4 are proved to be important transcription factors in a development process of the heart, and folic acid is found to have larger influence on the mef2c, the bmp2b, the vmhc, the has2 and the ephB4.

Human mef2 family contains four members including mef2a, mef2b, mef2c and mef2d, where the mef2b and the mef2c are activated in heart embryos at embryonic day 7, the mef2c is a key member in formation of primitive heart tube mesoderm cells during embryonic development, and an absence of the mef2c leads to severe cardiac structural abnormalities, particularly atrial septal defects or ventricular septal defects [Qiao XH, Wang F, Zhang XL, et al. MEF2C loss-of-function mutation contributes to congenital heart defects. Int J Med Sci. 2017; 14(11):1143-1153]. Folic acid affects an expression of the mef2c, which is also a possible reason for an increased birth rate of ASDs. In addition, hyaluronic acid is highly expressed in heart development and plays an important role in cell migration and transformation. Human *has* gene expression regulates hyaluronic acid synthesis. It has been reported that *has2* gene mutation may affect formation of the heart septum in embryos [Zhu X, Deng X, Huang G, et al. A novel mutation of Hyaluronan synthase 2 gene in Chinese children with ventricular septal defect. PLoS One. 2014; 9(2): e87437].

Zebrafish embryos under 5-methyltetrahydrofolate intervention at different times (24 h and 48 h) are collected and expressions of different transcription factors in the zebrafish embryos are detected by using a quantitative PCR techniques. As a result, under the 5-methyltetrahydrofolate intervention, an influence on the expressions of the transcription factors is not found and expressions of the relevant transcription factors are not different from those of a normal control group.

In one example, it is found that 5-methyltetrahydrofolate cannot save malformations of the zebrafish embryos caused by pericardium edema due to folic acid synthesis, but can save malformation performances of bradycardia and a short body length of zebrafish.

In order to further determine an influence of folic acid on development of embryonic heart, in example 9, the influence of the folic acid at different doses on the heart of a fetal rat is examined and results show that at a certain dose, in the heart of the fetal rat, a ventricular wall is too thin and ventricular septal defects can be seen individually; and in example 10, fetuses of maternal mice taking folic acid and maternal mice taking 5-methyltetrahydrofolate are compared, a congenital heart disease incidence of fetal mice is 5.95% in a low-dose folic acid group and 22.2% in a high-dose folic acid group, while no congenital heart disease is found in a 5-methyltetrahydrofolate group, such that an influence of folic acid on the development of the embryonic heart is further proved.

Furthermore, the 5-methyltetrahydrofolate can prevent embryonic heart teratogenesis induced by alcohol, lead nitrate and aristolochic acid A, and the substances respectively represent newborn heart malformations caused by different environmental factors such as alcoholism, heavy metals, medicines and the like, such that the 5-methyltetrahydrofolate has a wide effect of preventing congenital heart disease.

In one example of the present disclosure, the 5-methyltetrahydrofolate can prevent cardiac malformations caused by folate deficiency.

The medicine or health-care food of the present disclosure contains 0.1 mg-200 mg of the 5-methyltetrahydrofolate per dose and may preferably contain 0.1 mg-1 mg of the 5-methyltetrahydrofolate per dose when used for preventive administration. A specific dosage depends on various factors such as age, body weight, administration time and route, individual condition and the like of a patient, where an optimum dosage may preferably be 0.1-1 mg/day, for example, 0.8 mg/day.

In the present disclosure, the medicine or health-care food includes various carriers, excipients and/or auxiliary functional agents, such as water, oil, benzyl alcohol, polyethylene glycol, glycerin triacetate, gelatin, lecithin, cyclodextrin, lactobiose and starch, and magnesium stearate, talc, colloidal silica and cellulose. The auxiliary functional agents can be a stabilizer, an antioxidant, a buffer, an antimicrobial agent and the like. In one embodiment of the present disclosure, the excipients or carriers are microcrystalline cellulose, or a combination of microcrystalline cellulose and croscarmellose sodium or superfine silica powder.

It is found that after pregnant women take folic acid, unmetabolized folic acid entering blood circulation can cause congenital heart disease. The folic acid is a synthetic oxidized form not found in natural food and is required to be converted to tetrahydrofolic acid by a human dihydrofolate reductase to be used by humans, while the process is slow in humans. The unmetabolized folic acid can be detected in plasma several hours after folic acid administration of more than 200 µg, which is also a possible reason that clinical statistical results of an influence of folic acid on congenital heart disease are confused and a conclusion is inconsistent. The present disclosure also explains the reason of mutual contradiction of previous researches. A positive conclusion can be easily made in the 80-90s of the last century since policies of nutrition and folic acid supplementation are not proposed, and pregnant women may have a relatively low folic acid level. With increase of nutritional supplement-containing folic acid food, a relevance of folic acid and congenital heart disease is difficult to make under a large dose of folic acid. Furthermore, since an incidence of septal defects is very low before 2000, although folic acid is statistically found to have some correlation with this subtype, the authors of the study may neglect this phenomenon under cognitive deficits and biases or consider it as a statistical deviation.

Although folic acid has been used in a large scale to prevent congenital malformations of fetuses, the present disclosure discovers an increased incidence of congenital heart disease after folic acid supplementation by analyzing epidemiological data and regional folic acid supplementation results, and carefully suggests the folic acid supplementation increases an incidence of congenital heart disease. The conclusion is also proved by animal experiments and epigenetic results of the present disclosure.

The present disclosure also finds that 5-methyltetrahydrofolate has an effect of preventing congenital heart disease. The prophylactic dosage of the 5-methyltetrahydrofolate can be greater than 1 mg. Compared with synthetic folic acid, high-dose use has no teratogenic effect. Thus the 5-methyltetrahydrofolate can prevent birth defects in a high dose and has a significant progress in preventing neonatal congenital heart disease.

### BRIEF DESCRIPTION OF THE DRAWINGS

FIG. 1 shows a birth rate of different subtypes of congenital heart disease (CHD) in China from 1980 to 2019 and a birth rate of atrial septal defect (ASD) in different regions of the world from 1970 to 2017;
FIG. 2 shows a: an effect of 10 mM L-5-methyltetrahydrofolate (MTHF)-Ca on a survival rate of zebrafish embryos in example 1; and b: an effect of L-5-MTHF-Ca on development of zebrafish embryos in example 1;
FIG. 3 shows an effect of L-5-MTHF-Ca on heart rate and body length of zebrafish at 48 hpf and 72 hpf in example 1;
FIG. 4 shows a: an effect of methotrexate (MTX) and MTX+L-5-MTHF-Ca on a survival rate of zebrafish embryos in example 2; ns means no significant difference compared with a control group; ^{∗∗∗∗} means p < 0.005; and b: an influence of na MTX modeling group and a L-5-MTHF-Ca rescue group on development of zebrafish embryos;
FIG. 5 shows an effect of MTX and MTX+L-5-MTHF-Ca on a pericardium edema percentage, a heart rate and a body length of zebrafish embryos in example 2; ns means no significant difference compared with a control group; ^{∗∗∗∗} means p < 0.005; ^{∗∗∗} means p < 0.01; and ^{∗} means p < 0.1;
FIG. 6 shows a: an effect of folic acid at different concentrations on a survival rate of zebrafish embryos in example 3; and b: an effect of folic acid at different concentrations on a heart rate of zebrafish embryos;
FIG. 7 shows an effect of folic acid at different concentrations on embryo morphology of zebrafish embryos at 8 hpf and 24 hpf in example 3;
FIG. 8 shows an effect of 10 mM of L-5-MTHF-Ca, 8 mM of FA, 6 mM of folic acid (FA) and 4 mM of FA on zebrafish heart at 72 hpf in example 3; left: development of heart embryos influenced by different concentrations of folic acid is observed under a microscope at 72 hpf and pericardium edema malformation\atrium and ventricle elongation, and developmental disorder of lower intestinal network blood vessels are found; and right: an effect of folic acid on a body length of zebrafish at 72 hpf;
FIG. 9 shows an effect of folic acid on expressions of different transcription factors of zebrafish embryos in example 4;
FIG. 10 shows an effect of 5-methyltetrahydrofolate on expressions of different transcription factors of zebrafish embryos in example 4;
FIG. 11 shows a: an effect of FA and FA+L-5-MTHF-Ca on a survival rate of zebrafish embryos in example 5; and b: an effect of FA and FA+L-5-MTHF-Ca on development of zebrafish embryos in example 5;
FIG. 12 shows a pericardium edema percentage and a heart rate of zebrafish embryos at 48 hpf and a body length of zebrafish embryos at 72 hpf in example 5;
FIG. 13 shows a: an effect of formaldehyde (HCHO) and HCHO+L-5-MTHF-Ca on a survival rate of zebrafish embryos in example 6; and b: an effect of HCHO and HCHO+L-5-MTHF-Ca on development of zebrafish embryos;
FIG. 14 shows an effect on a heart rate of zebrafish embryos at 48 hpf and a body length of zebrafish embryos at 72 hpf in example 6;
FIG. 15 shows a: an effect of low- and medium-dose homocysteine on myocardial trabeculae in example 7; b: low-dose homocysteine leads to a small amount of lymphocyte infiltration in a myocardial interstitial space; c: low-dose homocysteine leads to a light pink serous material in the right ventricle; and d: high-dose homocysteine leads to red staining of fetal rat muscle cytoplasm;
FIG. 16 shows an effect of high-dose homocysteine on fetal rat heart in example 7;
FIG. 17 shows heart slices of rats in a normal group of example 8;
FIG. 18 shows an effect of high-dose folic acid (36.44 mg/kg) on fetal rat heart in example 8;
FIG. 19 shows an effect of high-dose folic acid (18.22 mg/kg) on fetal rat heart in example 8;
FIG. 20 shows an effect of high-dose folic acid (9.11 mg/kg) on fetal rat heart in example 8;
FIG. 21 shows typical heart slices of fetal rats in a 2.2775 mg/kg folic acid group in example 9;
FIG. 22 shows typical heart slices of fetal rats in a 0.911 mg/kg folic acid group in example 9;
FIG. 23 Echocardiogram of 6-day-old neonatal mice in example 10 including a control group (A), a low-dose folic acid group (B), a high-dose folic acid group (C), a low-dose methyltetrahydrofolate group (D) and a high-dose methylenetetrahydrofolate group (E);
FIG. 24 Echocardiogram data of 6-day-old neonatal mice in example 10 including a control group (A) Data, a low-dose folic acid group (B), a high-dose folic acid group (C), a low-dose methyltetrahydrofolate group (D) and a high-dose methylenetetrahydrofolate group (E);
FIG. 25 shows typical heart slices of fetal mice in each group of example 10;
FIG. 26 shows in example 11 a: a survival rate test of Pb(NO₃)₂ and Pb(NO₃)₂+L-5-MTHF-Ca on zebrafish embryos; and b: an effect of a Pb(NO₃)₂ modeling group and an L-5-MTHF-Ca rescue group on zebrafish embryonic development;
FIG. 27 shows an effect of lead acetate and methyltetrahydrofolate on a heart rate of zebrafish embryos at 48 hpf and a body length and a malformation rate of zebrafish embryos at 72 hpf in example 11;
FIG. 28 shows in example 11 a: ethanol (EtOH) and EtOH+L-5-MTHF-Ca on a survival rate of zebrafish; and b: an effect of EtOH and L-5-MTHF-Ca on development of zebrafish;
FIG. 29 shows an effect of EtOH and MTHF on a heart rate of zebrafish embryos and a body length and a malformation rate of zebrafish embryos at 48 hpf in example 11;
FIG. 30 shows a survival rate test of aristolochic acid A (AAA) and AAA+L-5-MTHF-Ca on zebrafish embryos in example 11;
FIG. 31 shows an effect of an AAA modeling group and L-5-MTHF-Ca+AAA group on development of zebrafish embryos example 11; and
FIG. 32 shows an effect of AAA and 5-methyltetrahydrofolate on a pericardium edema percentage and a heart rate of zebrafish embryos at 48 hpf and a body length of zebrafish embryos at 72 hpf in example 11.

### DETAILED DESCRIPTION

### Embodiment 1

0.4 g of calcium 5-methyltetrahydrofolate was mixed with 700 g of microcrystalline cellulose, a mixture was subjected to dry granulation and 1,000 capsules were filled to prepare a capsule preparation containing 0.4 mg of 5-methyltetrahydrofolate calcium each.

### Example 2

1 g of calcium 5-methyltetrahydrofolate was added to 200 g of superfine silica powder to be mixed well, a mixture was pressed into a tablet containing 2 mg of 5-methyltetrahydrofolate each by a tablet machine.

### Example 1 Effect of calcium 5-methyltetrahydrofolate (L-5-MTHF-Ca) on survival rate and cardiovascular development of zebrafish embryos

Transgenic zebrafish (fli-1:EGFP) were from the Model Animal Research Center of Nanjing University. Adult zebrafish aged less than 1 were used for experiment and fed at a water pH value of 7±0.2, a temperature around 28°C and a ratio of illumination to darkness of 14 h:10 h, and with *Artemia salina* eggs twice a day. Three zebrafish, one female, two males, were placed in a spawning box the night before and eggs were collected the next morning. Fish eggs were placed in an embryo culture medium (prepared with 0.2 g/L of sea salt) and cultured at 28°C. Zebrafish embryos were cultured in 24-well plates with 10 embryos per well (n=10) and 1 mL of the embryo culture medium was added in advance. Each experiment was conducted in triplicate. Zebrafish embryos were administrated at 2 h post fertilization (2 hpf) and termination was conducted at 72 hpf. The number of deaths in each group was recorded at 8, 24, 48 and 72 hpf. No food was fed during a dosing period, and the dead embryos were cleaned up in time. A phenotype of resulted malformation was observed and the number of all abnormal zebrafish where the malformation was present was recorded. A survival rate, morphological defects, a heart rate, cardiac morphology and other indicators were observed and evaluated by an SMZ745T inverted stereomicroscope.

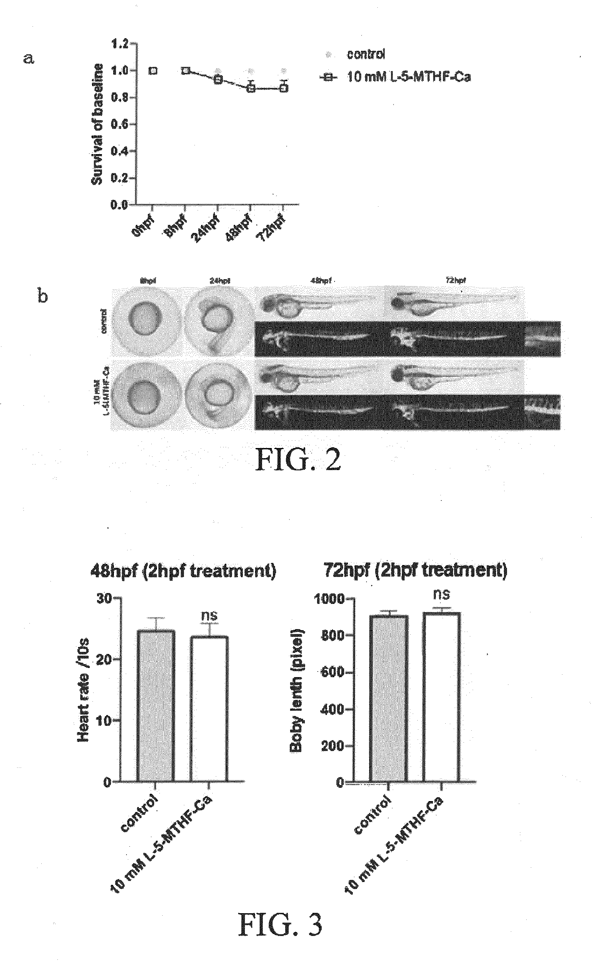

Test results were as follows: it was found that 10 mML of L-5-MTHF-Ca did not affect an embryo survival rate. Compared with a normal group at 8 hpf, normal embryos had a similar phenotype with embryos treated with 10 mML of L-5-MTHF-Ca, and embryos were almost completely covered. At 24 hpf, the normal embryos were similar to the embryos treated with 10 mML of L-5-MTHF-Ca, and developed head and tail, and the tails swung. After 48 hpf, the head and the tail of each group were fully developed, the whole body pigment was formed, the heart beat was obvious, the intersegmental blood vessels developed normally, and blood flowed throughout the body. At 72 hpf, the heart in each group had a normal shape and the lower intestinal network blood vessels developed normally (FIG. 2). A heart rate of zebrafish embryos was measured at 48 hpf, a body length of the zebrafish embryos was measured at 72 hpf and it could be found that a group treated with 10 mML of L-5-MTHF-Ca had no difference in the heart rate and the body length from the control group (FIG. 3).

### Example 2 Effect of L-5-MTHF-Ca on cardiovascular development system of folic acid-deficient zebrafish embryo model induced by methotrexate (MTX)

Transgenic zebrafish (fli-1:EGFP) were from the Model Animal Research Center of Nanjing University. Adult zebrafish aged less than 1 were used for experiment and fed at a water pH value of 7±0.2, a temperature around 28°C and a ratio of illumination to darkness of 14 h:10 h, and with *Artemia salina* eggs twice a day. Three zebrafish, one female, two males, were placed in a spawning box the night before and eggs were collected the next morning. Fish eggs were placed in an embryo culture medium (prepared with 0.2 g/L of sea salt) and cultured at 28°C. Zebrafish embryos were cultured in 24-well plates with 10 embryos per well at 6 hpf in a dosing manner, 1 mL of a 1.5 mM MTX solution was added, termination was conducted at 10 hpf, and the zebrafish embryos were transferred to an egg solution for continuous culture to 72 hpf. Dead embryos were cleaned up in time. A phenotype of malformation was observed, the number of all abnormal zebrafish where the malformation was present was recorded and a malformation rate and a rescue rate of each group were calculated. Morphological defects, a heart rate, cardiac morphology and other indicators were observed and evaluated by an SMZ745T inverted stereomicroscope.

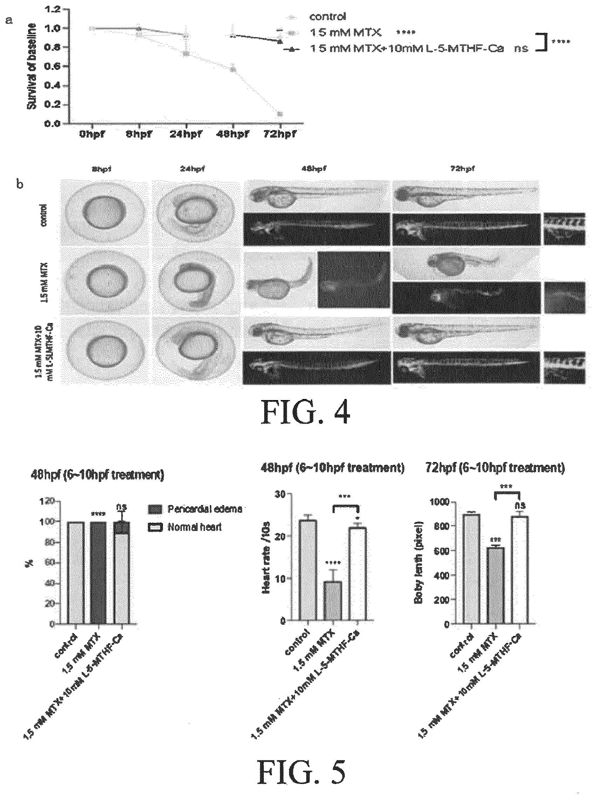

The results were as follows (FIG. 4) and it was found that 1.5 mM of MTX can seriously lead to a low survival rate of embryos (the survival rate was lower than 0.1), while 10 mM L-5-MTHF-Ca can rescue an effect of 1.5 mM MTX on a survival rate of embryos, which had no significant difference from that of a control group. Compared with the normal control group, phenotypes of zebrafish embryos at different time points were evaluated and zebrafish embryos treated with 1.5 mM MTX or 1.5 mM MTX+10 mM L-5-MTHF-Ca did not show obvious embryonic developmental delay; 1.5 mM MTX led to hypoplasia and deformities such as small heads and short tails of zebrafish at 24 hpf; 1.5 mM MTX led to body bending, intersegmental vascular hypoplasia and pericardium edema of zebrafish at 48 hpf; and 1.5 mM MTX led to a short body length and lower intestinal network vessel hypoplasia at 72 hpf. In contrast, 10 mM L-5-MTHF-Ca could rescue embryonic development malformations induced by 1.5 mM MTX. A malformation rate was 100% in a MTX group, but only 10% after adding L-5-MTHF-Ca.

To further quantitatively assess a rescue effect of 10 mM L-5-MTHF-Ca on MTX, pericardium edema and a 10 s heart rate of zebrafish embryos were assessed at 48 hpf, and a body length of zebrafish among groups was assessed at 72 hpf. It can be seen from FIG. 5, 1.5 mM MTX seriously led to pericardium edema, bradycardia, and a shortened body length of zebrafish, while 10 mM L-5-MTHF-Ca could rescue malformed phenotypes of the pericardium edema, the bradycardia and the shortened body length.

### Example 3 Teratogenic effect of folic acid (FA) on zebrafish embryos

Transgenic zebrafish (fli-1:EGFP) were from the Model Animal Research Center of Nanjing University. Adult zebrafish aged less than 1 were used for experiment and fed at a water pH value of 7±0.2, a temperature around 28°C and a ratio of illumination to darkness of 14 h:10 h, and with *Artemia salina* eggs twice a day. Three zebrafish, one female, two males, were placed in a spawning box the night before and eggs were collected the next morning. Fish eggs were placed in an embryo culture medium (prepared with 0.2 g/L of sea salt) and cultured at 28°C. Zebrafish embryos were cultured in 24-well plates with 5 embryos (n=5) per well at 2 hpf in a dosing manner, 1 mL of a 20 mM NaHCO₃ solution, 1 mL of a 10 mM FA solution (20 mM NaHCO₃ as a solvent), 1 mL of a 8 mM FA solution (20 mM NaHCO₃ as a solvent), 1 mL of a 6 mM FA solution (20 mM NaHCO₃ as a solvent), 1 mL of a 4 mM FA solution (20 mM NaHCO₃ as a solvent), 1 mL of a 2 mM FA ((20 mM NaHCO₃ as a solvent) and 1 mL of 10 mM L-5-MTHF-Ca (20 mM NaHCO₃ as a solvent) were separately added to each well, and termination was conducted at 72 hpf. Dead embryos were cleaned up in time. Each experiment was conducted in triplicate. Dead embryos were cleaned up in time. A phenotype of malformation was observed, the number of all abnormal zebrafish where the malformation was present was recorded and a malformation rate and a rescue rate of each group were calculated. Morphological defects, a heart rate, cardiac morphology and other indicators were observed and evaluated by an SMZ745T inverted stereomicroscope.

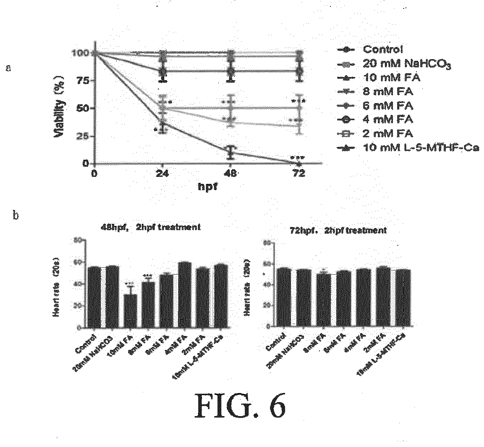

The results were as follows and it was found that 20 mM NaHCO₃ did not affect a survival rate of zebrafish, 10 mM L-5-MTHF-Ca did not affect the survival rate of zebrafish, while all FA groups showed a low survival rate of embryos, with an increased dose of folic acid, a mortality of the zebrafish embryos also increased (FIG. 6), and the folic acid also affected an embryonic heart rate, especially at 48 hpf. The folic acid also affected a body length of zebrafish embryos, especially at 72 hpf and the folic acid at a concentration of 8 mM significantly shortened the body length of the embryos.

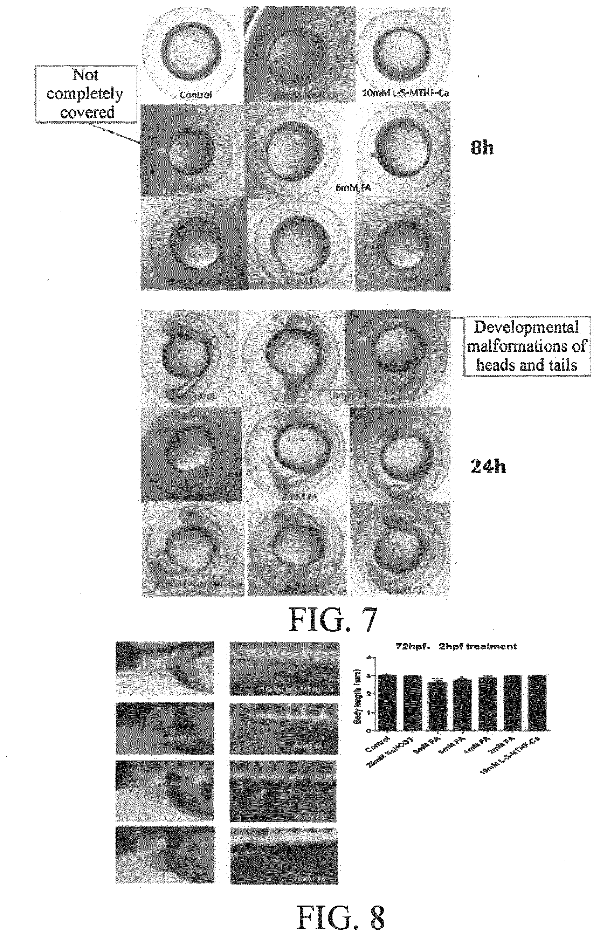

Compared with a normal control group, phenotypes of the zebrafish embryos at different time points were evaluated. At 8 hpf, the zebrafish embryos in a normal control group, a L-5-MTHF-Ca group, a NaHCO₃ group and a 2 mM FA group were all covered completely, while the zebrafish embryos of FA groups of other concentrations were all not covered completely and the folic acid was found to lead to developmental malformation of heads and tails of the embryos at 24 hpf (FIG. 7). At 72 hpf, the zebrafish embryos in the normal zebrafish group, the L-5-MTHF-Ca group, the NaHCO₃ group and the 2 mM FA group had a normal heart shape and normal development of lower intestinal network blood vessels, while in the FA groups with concentrations of 4 mM or above, the zebrafish embryos showed pericardium edema malformations, atrial and ventricular elongation and developmental disorders of lower intestinal network blood vessels (FIG. 8).

### Example 4 Effect of folic acid (FA) on expressions of different transcription factors during development of zebrafish embryos

In order to explore an effect of folic acid on epigenetics during development of the zebrafish embryos, the zebrafish embryos at different developmental stages were collected for testing. Transgenic zebrafish (fli-1:EGFP) were from the Model Animal Research Center of Nanjing University. Adult zebrafish aged less than 1 were used for experiment and fed at a water pH value of 7±0.2, a temperature around 28°C and a ratio of illumination to darkness of 14 h:10 h, and with *Artemia salina* eggs twice a day. Three zebrafish, one female, two males, were placed in a spawning box the night before and eggs were collected the next morning. Fish eggs were placed in an embryo culture medium (prepared with 0.2 g/L of sea salt) and cultured at 28°C. Zebrafish embryos were cultured in 24-well plates with 10 per well (n=10) at 2 hpf in a dosing manner, 1 mL of a 8 mM FA solution (20 mM NaHCO₃ as a solvent) and 1 ml of 8 mM L-5- MTHF-Ca (20 mM NaHCO₃ as a solvent) were separately added into each well at 24 hpf and 48 hpf, and the zebrafish embryos were fixed overnight with 4% paraformaldehyde solution, stored in a methanol solution and placed at -20 °C for standby use. The zebrafish embryos were administrated at 2 hpf, 10 zebrafish with obvious phenotypes in each group were collected every 24 h, RNA of each group of zebrafish was extracted by Trizol according to the instructions, and the embryos from 2 time points in each group were extracted at 24 hpf and 48 hpf for a follow-up experiment.

A fluorescent dye SYBR Green was used in the qRT-PCR experiment. The qRT-PCR was conducted according to the instructions of an SYBR Green qPCR Master Mix kit and a detection was conducted on an ABI (HT 7900) PCR instrument. Gene primers were synthesized by GENEWIZ Biotechnology Co., Ltd. and specific information was shown in Table 1. An expression of β-actin was used as an internal reference and relative expression levels of other genes were calculated by a ΔΔCt method.

**Table 1 Sequences of primers for target genes and a reference gene**

| Target gene | Sequence of primers (5'-3') | |
|---|---|---|
| *β-actin* | F: | CGAGCAGGAGATGGGAACC |
| | R: | CAACGGAAACGCTCATTGC |
| *amhc* | F: | AAGGTAAAATCCTACAAACGTTCGG |
| | R: | CAAACAAATCAAAGTGCGATTGCAC |
| *vmhc* | F: | ACATAGCCCGTCTTCAGGATTTGG |
| | R: | GAGAGAAAGGCAAGCAAGTACTGG |
| *hand2* | F: | ACTCCGTCTGTGGTTCGC |
| | R: | TTGATGCTCTGGGTCCTG |
| *Nkx2.5* | F: | TTCAATCCAGCAGTGTTCCTTCA |
| | R: | ACATCCCAGCCAAACCATATCTC |
| *has2* | F: | TGGATGCAGGTTTGTGATTC |
| | R: | CTCCTCCAACATTGGGATCT |
| *mef2a* | F: | GAACCGGCAGGTTACCTTTA |
| | R: | GGGCAATCTCACAGTCACAC |
| *mef2c* | F: | AATCCGAGGACAAATATCGC |
| | R: | TTAGACTGAGGGATGGCACA |
| *bmp2b* | F: | CTTCCTCCTCCGAGGCTT |
| | R: | ACTGGCATCTCCGAGAACTT |
| *flk-1* | F: | GATGACCTGAAGACGCTGAA |
| | R: | CCAGCAGAACTGACTCCTTAC |
| *ephB4* | F: | TTCACCTGGAGGGCATAATAAC |
| | R: | CAGCATCCCGACTAACTGTATC |
| *eprinB2* | F: | CCGAGCGACATCATCATCC |
| | R: | TGTAAACAGGGTGTCCGTAATC |

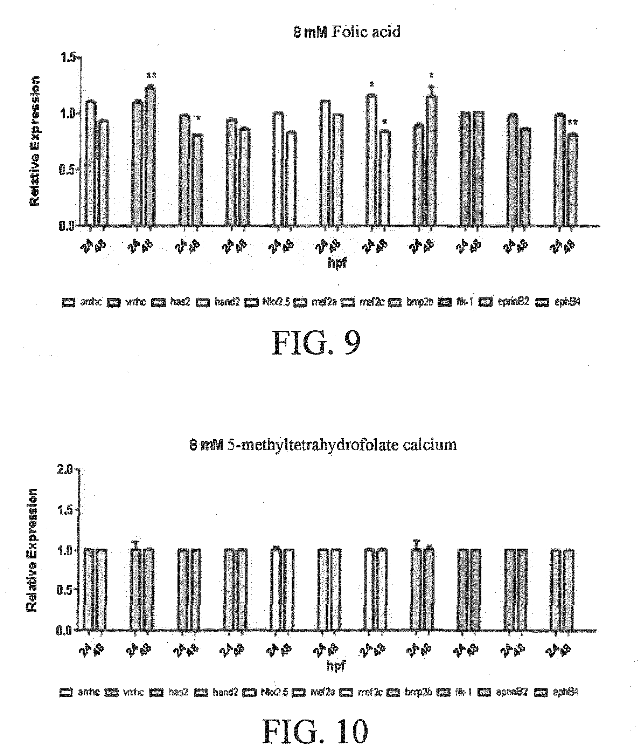

The results were as follows: zebrafish eggs formed a complete heart 48 h after fertilization and the heart consisted of atria and ventricles with valves between them. Under action of signal molecules, cardiomyogenic genes of cardiomyogenic cells were expressed and included *Nkx-2* family genes, *Mef2* family genes and the like. It was found that folic acid interfered with expressions of various genes and affected the expressions of the various genes to varying degrees compared with the normal control group (FIG. 9). However, the 5-methyltetrahydrofolate did not affect the expressions of the various genes compared with the normal control group (FIG. 10). It was believed that folic acid deficiency was related to development of congenital heart disease, but folic acid itself was also related to development of congenital heart disease. It was found that folic acid increased an expression of a *mef2c* gene and decreased an expression of a *bmp2b* gene at 24 hpf, while inhibited expressions of *has2, mef2c* and *ephb4* genes and increased an expression of a *vmhc* gene at 48 hpf. While the *mef2c* is a key member of primordial cardiac tube germ layer cells during embryonic development and the *has2* also affected formation of the embryonic cardiac septum, which is a possible reason for a significant increase of an ASD birth rate.

### Example 5 L-5-MTHF-Ca rescues effects of FA on teratogenesis, survival rate, heart rate and body length of zebrafish embryos

To investigate whether 5-methyltetrahydrofolate can reduce teratogenicity of FA, the following experiment was conducted. Transgenic zebrafish (fli-1:EGFP) were from the Model Animal Research Center of Nanjing University. Adult zebrafish aged less than 1 were used for experiment and fed at a water pH value of 7±0.2, a temperature around 28°C and a ratio of illumination to darkness of 14 h:10 h, and with *Artemia salina* eggs twice a day. Three zebrafish, one female, two males, were placed in a spawning box the night before and eggs were collected the next morning. Fish eggs were placed in an embryo culture medium (prepared with 0.2 g/L of sea salt) and cultured at 28°C. Zebrafish embryos were cultured in 24-well plates with 10 embryos per well at 2 hpf in a dosing manner, 1 mL of a 6 mM FA solution was added (20 mM NaHCO₃ as a solvent), and termination was conducted at 72 hpf. Dead embryos were cleaned up in time. Each experiment was conducted in triplicate. 6 mM FA was selected for modeling, 10 mM L-5-MTHF-Ca was used for the rescue experiment, and 20 mM NaHCO₃ as a solvent was set as a control group. A phenotype of malformation was observed, the number of all abnormal zebrafish where the malformation was present was recorded and a malformation rate and a rescue rate of each group were calculated. Morphological defects, a heart rate, cardiac morphology and other indicators were observed and evaluated by an SMZ745T inverted stereomicroscope.

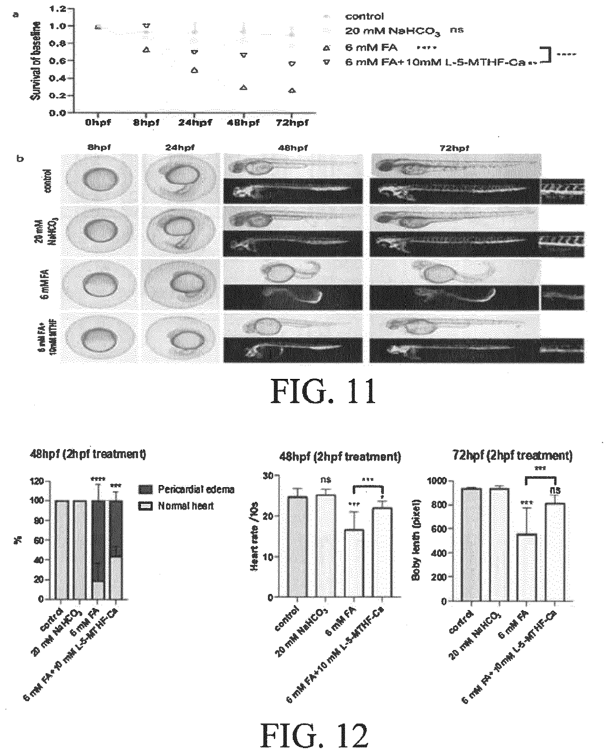

6 mM FA was selected for modeling, 10 mM L-5-MTHF-Ca was used for the rescue experiment, and 20 mM NaHCO₃ as a solvent was set as the control group. The results were as follows: 20 mM NaHCO₃ did not affect a survival rate of zebrafish, while 6 mM FA severely led to a low survival rate of zebrafish, but the 10 mML-5-MTHF-Ca can rescue an effect of the 6 mM FA on a survival rate of the embryos. Compared with the normal control group, phenotypes of the zebrafish embryos at different time points were evaluated (FIG. 11). At 8 hpf, the normal embryos were almost completely covered, while 6 mM FA and 6 mM FA+10 mM L-5-MTHF-Ca delayed development of the zebrafish embryos. At 24 hpf, the normal zebrafish embryos developed heads and tails, and the tails swung. 6 mM FA led to small heads and short tails of zebrafish, while 6 mM FA+10 mM L-5-MTHF-Ca alleviated the malformation. At 48 hpf, both 6 mM FA and 6 mM FA+10 mM L-5-MTHF-Ca led to pericardium edema, dyspigmentation, and intersegmental vascular hypoplasia of zebrafish. At 72 hpf, 6 mM FA and 6 mM FA+10 mM L-5-MTHF-Ca led to developmental disorders of lower intestinal network blood vessels of the zebrafish. However, the zebrafish embryos in a 20 mM NaHCO₃ group (a solvent control group) had a phenotype similar to that of the normal group observed at various time points.

To further quantitatively assess a rescue effect of the 10 mM L-5-MTHF-Ca on 6 mM FA, pericardium edema and a 10 s heart rate of zebrafish embryos were assessed at 48 hpf, and a body length of zebrafish among groups was assessed at 72 hpf. It can be seen from FIG. 12, the 6 mM FA seriously led to pericardium edema of zebrafish, while the 10 mM L-5-MTHF-Ca could not rescue the pericardium edema; and the 6 mM FA seriously led to bradycardia and a shortened body length of zebrafish, while the 10 mM L-5-MTHF-Ca could rescue malformed phenotypes of the bradycardia and the shortened body length. 20 mM NaHCO₃ (a solvent control group) had no effect on the heart, the heart rate and the body length of the zebrafish embryos.

In conclusion, 10 mM L-5-MTHF-Ca could rescue 6 mM FA-induced reduced survival rate, bradycardia, and shortened body length of the zebrafish embryos, but the not pericardium edema and the dyspigmentation.

### Example 6 L-5-MTHF-Ca rescues effect of formaldehyde on survival rate of zebrafish embryos

Transgenic zebrafish (fli-1:EGFP) were from the Model Animal Research Center of Nanjing University. Adult zebrafish aged less than 1 were used for experiment and fed at a water pH value of 7±0.2, a temperature around 28°C and a ratio of illumination to darkness of 14 h:10 h, and with *Artemia salina* eggs twice a day. Three zebrafish, one female, two males, were placed in a spawning box the night before and eggs were collected the next morning. Fish eggs were placed in an embryo culture medium (prepared with 0.2 g/L of sea salt) and cultured at 28°C. Zebrafish embryos were cultured in 24-well plates with 10 embryos per well at 2 hpf in a dosing manner (10 mM L-5-MTHF-Ca) and 1 mL of a 30 mM HCHO solution was added. Dead embryos were cleaned up in time. Each experiment was conducted in triplicate. A phenotype of malformation was observed, the number of all abnormal zebrafish where the malformation was present was recorded and a malformation rate and a rescue rate of each group were calculated. Morphological defects, a heart rate, cardiac morphology and other indicators were observed and evaluated by an SMZ745T inverted stereomicroscope.

The results were as follows: 30 mM FA was selected for modeling, 10 mM L-5-MTHF-Ca was used for the rescue, 30 mM HCHO would severely lead to a low survival rate of embryos, but the 10 mML-5-MTHF-Ca can rescue an effect of the 30 mM HCHO on a survival rate of the embryos.

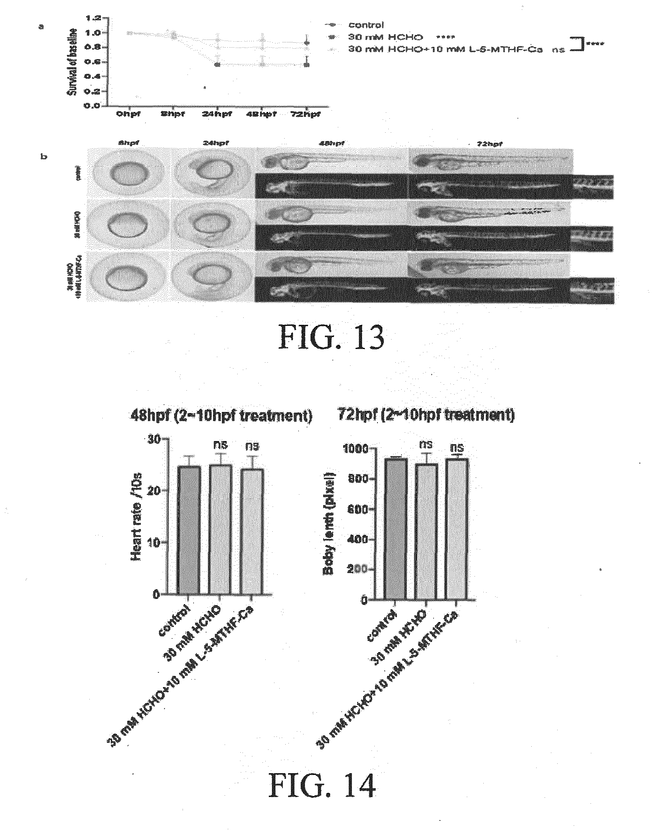

Compared with the control group, phenotypes of the zebrafish embryos at different time points were evaluated (FIG. 13). At 8 hpf, the normal embryos were almost completely covered, while the 30 mM HCHO and 30 mM HCHO+10 mM L-5-MTHF-Ca delayed development of the zebrafish embryos. At 24 hpf, the normal zebrafish embryos developed heads and tails, and the tails swung. The zebrafish treated with the 30 mM HCHO and 30 mM HCHO+10 mM L-5-MTHF-Ca had phenotypes similar to those of the zebrafish in the control group. At 48 hpf, the zebrafish embryos were observed and photographed with a brightfield fluorescence microscope. The normal embryos were similar to the embryos treated with the 30 mM HCHO and the 30 mM HCHO+10 mM L-5-MTHF-Ca, the head and the tail were fully developed, the whole body pigment was formed, the heart beat was obvious, the intersegmental blood vessels developed normally, and blood flowed throughout the body. At 72 hpf, the normal embryos and the embryos treated with 30 mM HCHO and 30 mM HCHO+10 mM L-5-MTHF-Ca had a normal heart shape and normal development of lower intestinal network blood vessels.

To further quantitatively assess a rescue effect of the 10 mM L-5-MTHF-Ca on 30 mM HCHO, pericardium edema and a 10 s heart rate of zebrafish embryos were assessed at 48 hpf, and a body length of zebrafish among groups was assessed at 72 hpf. It can be seen from FIG. 14 that 30 mM HCHO and 30 mM HCHO+10 mM L-5-MTHF-Ca had no effect on the pericardium edema, the heart rate and the body length of the zebrafish.

In conclusion, the 30 mM HCHO was lethal but not teratogenic to zebrafish and 10 mM L-5-MTHF-Ca could rescue a reduced survival rate of the zebrafish embryos caused by the 30 mM HCHO. The finding suggested that the L-5-MTHF-Ca could rescue a high mortality rate of embryos in pregnant women exposed to formaldehyde for a long time.

### Example 7 Effects of homocysteine on fetal rats

In order to investigate an effect of homocysteine on the fetal heart, SD rats were selected, male and female rats were mated in a cage at a ratio of 1:1 overnight, a vaginal plug was checked the next morning, a day when the vaginal plug was found was regarded as day 0 of pregnancy, and pregnant mice were fed alone. A 1% homocysteine (HCY) solution was used, an injection volume was calculated according to an injection volume of 1 ml/100 g, a dose was (100 mg/kg/d), on the seventh day of pregnancy, the pregnant mice were intraperitoneally injected once a day until the 17th day of pregnancy and injected once on the 19th day of pregnancy, no fluid was withdrawn during each injection, the pregnant mice were subjected to chloral hydrate anesthesia on the 20th day of pregnancy, and a fetus was obtained by cesarean section. High-dose HCY model pregnant mice were investigated by the same method as above, the dose was 200 mg/kg/d and the other conditions were the same. Fetal rats were dissected, the heart was taken and sliced for observation, and the results were as follows.

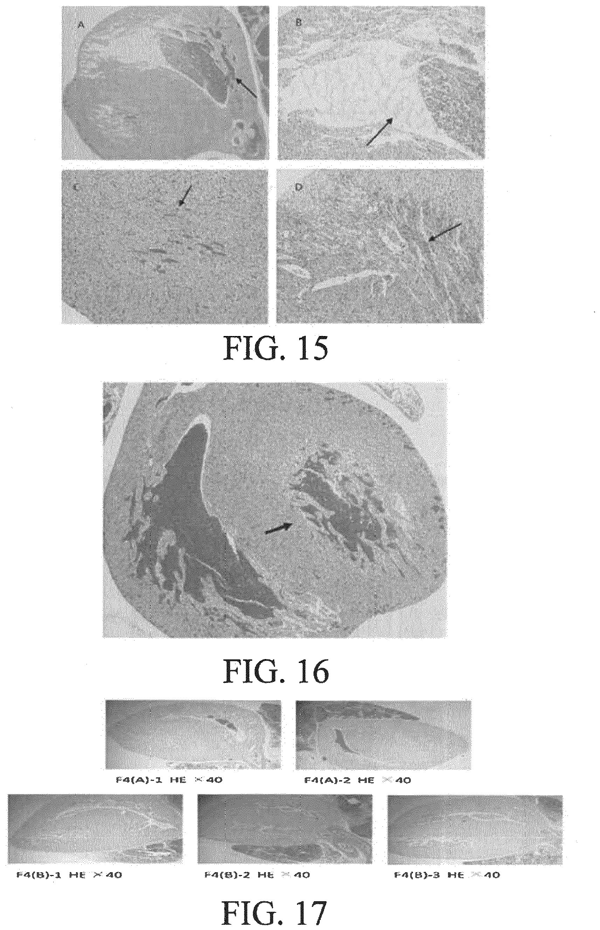

Low-dose HCY led to widening of myocardial trabeculae in fetal rats and atria was filled with red blood cells (FIG.15a). There was a small amount of lymphocytic infiltration in a myocardial space (FIG. 15b). There was a small amount of lymphocytic infiltration in a right myocardial space (FIG. 15c). High-dose HCY led to red staining of muscle cytoplasm of the fetal rats as shown by black arrows (FIG. 15d). The ventricles and atria were filled with the red blood cells and the myocardial trabeculae were widened (FIG. 16). The above experiment showed that the HCY could affect the fetal rat heart and it was speculated that a high level of the homocysteine in humans may also lead to congenital heart disease.

### Example 8 Effect of high-dose folic acid on fetal rat heart (pre-experiment)

28 each of female and male adult healthy SD rats, weighing about 200-250 g, were purchased from SPF (Beijing) biotechnology co., LTD. After all animals were purchased, general physiological indicators, body weight and feeding of the animals were observed. The animals were adaptively fed for one week. The animals were fed with a standard pelleted feed and had a free access to water. The animals were fed day and night in natural lighting, and at a room temperature of 18-26°C and a relative humidity of 40%-70%.

The obtained 16 pregnant mice were randomly divided into 4 groups with 4 rats in each group according to body weight: a normal group, a high-dose folic acid group, a middle-dose folic acid group and a low-dose folic acid group. The high-dose folic acid (36.44 mg/kg) group, the middle-dose folic acid (18.22 mg/kg) group, the low-dose folic acid (9.11 mg/kg ) group and a blank control group were separately set. The pregnant mice were intragastrically administrated with a 1 ml/100 g solution. The pregnant mice were given pure water in the normal group. The pregnant mice in the other groups started to be administrated on the 2nd day after grouping once a day for 21 consecutive days. The pregnant mice in each group freely ate and drunk. After the female rats were administrated for 7 days, male and female rats were mated in a cage at a ratio of 1:1 overnight, a vaginal plug was checked the next morning, a day when the vaginal plug was found was regarded as day 0 of pregnancy, the pregnant mice were separately fed and administrated for 21 consecutive days, and a fetus was obtained by cesarean section.

Routine histopathologic paraffin sample preparation, serial section (a thickness of 5 µm) and HE staining were conducted. Sample preparation: routine histopathologic paraffin sample sections were prepared and each sample was 1 paraffin block; and section: each numbered sample had 20 sections. A starting point of mounting was when the paraffin block was sliced, there were ventricular mounting slices and 4 consecutive tissue points were mounted on each glass slide on average. HE staining: there were 20 glass slides for each sample and every other 1 slide was taken for HE staining, a total of 10 slides. Observation and analysis: Leica panoramic image acquisition and comparative analysis were conducted. Fetal rat hearts were dissected and sliced for observation, and the results were as follows.

The atrium and the ventricle of the rats in the normal group were well differentiated, and the pericardium, endocardium and epicardium were intact. Longitudinal, oblique, or transverse sections of myocardial fibers could be observed simultaneously. Myocardial fibers were neat and regular with clear texture, and the muscle fibers were branched and anastomosed into a network. The myocardial fibers were divided into fiber bundles of different sizes by loose connective tissues and there were abundant blood vessels in the bundles. Myocardial trabeculae were densely developed in the atrium and the ventricle. Cardiomyocytes were normal and nuclei were neatly arranged, oval or spherical, and subjected to light blue staining. There were a small amount of round or oval red-stained blood cells between the myocardial fibers. No obvious pathological changes showed. Due to development or cutting angles, an aortic structure connected to the ventricles was not shown in some animals (FIG. 17).

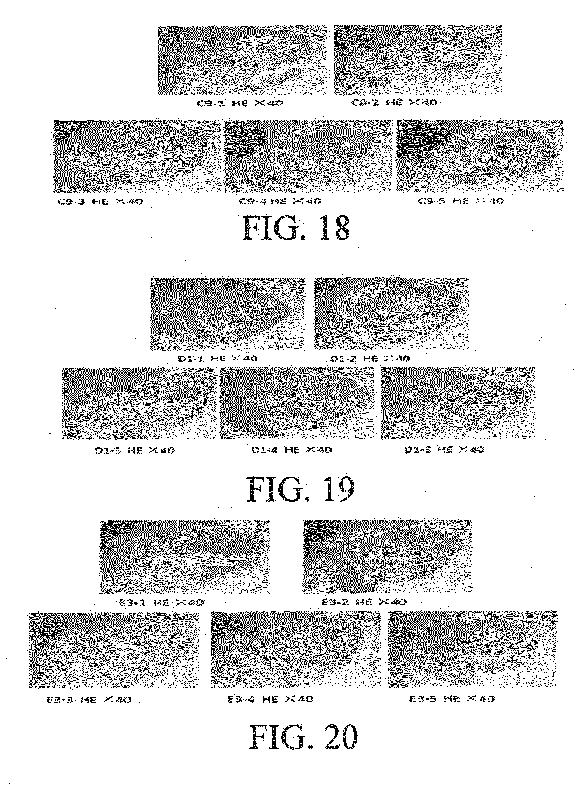

In the high-dose folic acid group, most animals had delayed cardiac development (n=3), a smaller heart size and thinner heart walls (C9-1, C9-4 and C9-5). In some animals, ventricular septum disappeared (n=1), right ventricule was defective (n=4), and the heart was a solid muscle tissue or only had a small cavity and almost did not have myocardial trabeculae; an inner wall of a left ventricular cavity did not have myocardial trabeculae or only had a small amount of short and thick myocardial trabeculae; and the left and right atria were basically normal (C9-2, C9-3, C9-4 and C9-5). The hearts of individual animals showed different microscopic features, left and right atrial cavities were obviously dilated, left and right ventricular cavities had a similar size, the left ventricle had sparse myocardial trabeculae, and the right ventricle had a smooth inner wall with almost no myocardial trabeculae (C9-1). Due to development or cutting angles, an aortic structure connected to the ventricles was not shown in some animals (C9-3 and C9-4) (FIG. 18).

In the middle-dose folic acid group, the ventricle and the atrium were differentiated, and the intact pericardium, endocardium and epicardium were seen. Longitudinal, oblique, or transverse sections of myocardial fibers could all be seen. The myocardial fibers were well developed and regularly arranged and had a clear texture. No degeneration and necrosis of histiocytes or infiltration of inflammatory cells were seen. However, the ventricle and the atrium of individual animals showed a certain degree of developmental delay (n=1) and the atrium and the ventricle had a relatively small volume (D1-3). In some animals, the ventricular cavity was narrow, the differentiation was delayed, the ventricular wall was smooth, and even ventricular defect existed (n=2), the ventricular cavity was almost invisible and a solid muscle tissue, the myocardial trabeculae were short and sparse in the ventricule (D1-3 and D1-5), and the myocardial trabeculae were sparse in the atrium (D1-3) (FIG. 19).

In the low-dose folic acid group, the differentiation of the atrium and the ventricle was completed, the myocardial fibers were neatly arranged in bundles, different sections of the myocardial fibers were visible, the texture was clear, and there were abundant capillaries between the myocardial fibers. However, the ventricular differentiation of some animals was delayed and the right ventricule was defective (n=3) with only a small cavity; and the left and right atrial cavities were slightly dilated and basically normal (E3-3 and E3-4). Individual animals showed obviously delayed cardiac development (n=1), the heart was smaller, the ventricular septum disappeared, and the right ventricule was defective and a solid muscle tissue, and no myocardial trabeculae were not found; and the left ventricular cavity became smaller, only had a small amount of myocardial trabeculae in the inner wall; and left and right atrial atrophy became smaller (E3-5) (FIG. 20).

The statistical data were shown in Table 2 below. It can be seen from the figure that folic acid may also affect a litter rate of rats and reduce the litter rate under a high dose.

**Table 2 Effects of folic acid on development of fetal rats**

| | Development delay | Ventricular defects/septal defects | Sparse myocardial trabeculae | Malformation cases | Malformation rate | Number of fetal rats |
|---|---|---|---|---|---|---|
| Folic acid group (36.44 mg/kg) | 3 | **4** | **1** | **4** | 25% | 16 |
| Folic acid group (18.22 mg/kg) | **1** | **2** | **2** | **4** | 15.4% | 26 |
| Folic acid group (9.11 mg/kg) | **1** | 3 | **1** | 3 | 10.7% | 28 |
| Blank control group | 0 | 0 | 0 | 0 | 0% | 26 |

### Example 9 Effect of folic acid on fetal rat heart under pharmacological dose

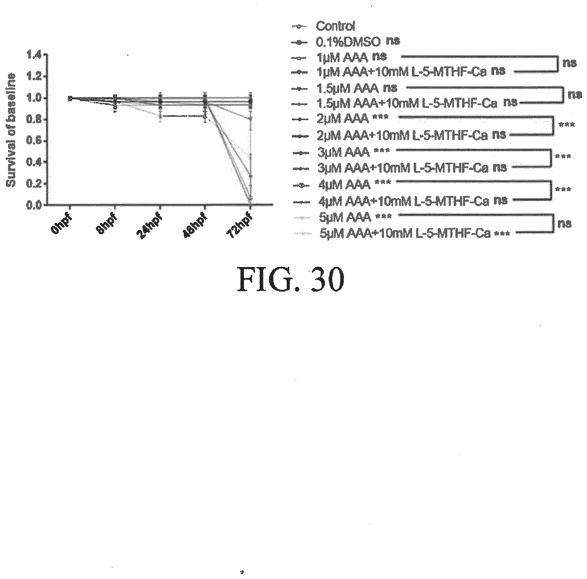

In order to investigate an effect of folic acid on fetal rat heart under pharmacological dose, 6 folic acid groups and a blank control group were divided in the experiment, the folic acid groups were divided into a folic acid group 1 (4.555 mg/kg/d), a folic acid group 2 (2.2775 mg/kg/d), a folic acid group 3 (0.911 mg/kg/d), a folic acid group 4 (0.4555 mg/kg/d), a folic acid group 5 (0.22775 mg/kg/d) and a folic acid group 6 (0.113875 mg/kg/d). The folic acid groups and the blank control group consisted of 4 pregnant mice each. The pregnant mice in the folic acid group 1 (4.555 mg/kg/d), the folic acid group 2 (2.2775 mg/kg/d) and the folic acid group 3 (0.911 mg/kg/d) were intraperitoneally injected, the pregnant mice in the folic acid group 4 (0.4555 mg/kg/d), the folic acid group 5 (0.22775 mg/kg/d) and the folic acid group 6 (0.113875 mg/kg/d) were intragastrically administrated, and the pregnant mice in the blank control group were naturally fed. After the female rats were administrated for 7 days, male and female rats were mated in a cage at a ratio of 1:1 overnight, a vaginal plug was checked the next morning, a day when the vaginal plug was found was regarded as day 0 of pregnancy, the pregnant mice were separately fed and administrated for 21 consecutive days, a fetus was obtained by cesarean section, and the number of fetal rats in each group was shown in FIG. 30. Fetal rat hearts were taken for a pathological examination and the results were as follows.

In the folic acid group 1 (4.555 mg/kg/d), the atrium and the ventricle were basically formed by differentiation, and no obvious pathological changes were found in pericardium, endocardium and myocardium. The left and right atriums were basically normally developed, a section of the right atrium was generally slightly larger than that of the left atrium, and abundant myocardial trabeculae could be seen in an inner wall of the cavity. Several individuals had smaller atrial cavities. The left and right ventricles were basically formed, the ventricular wall had rich blood vessels, the left ventricular wall was often thick and had a narrow cavity, there was no obvious left ventricular cavity (n=3), or only closely arranged myocardial trabeculae was seen. Several individuals also showed narrow cavities in the right ventricles. Several individuals also showed too thin right ventricular walls. Large blood vessel lumen connected to the ventricles could be seen in all sections and valves in the lumen could be seen in some sections.

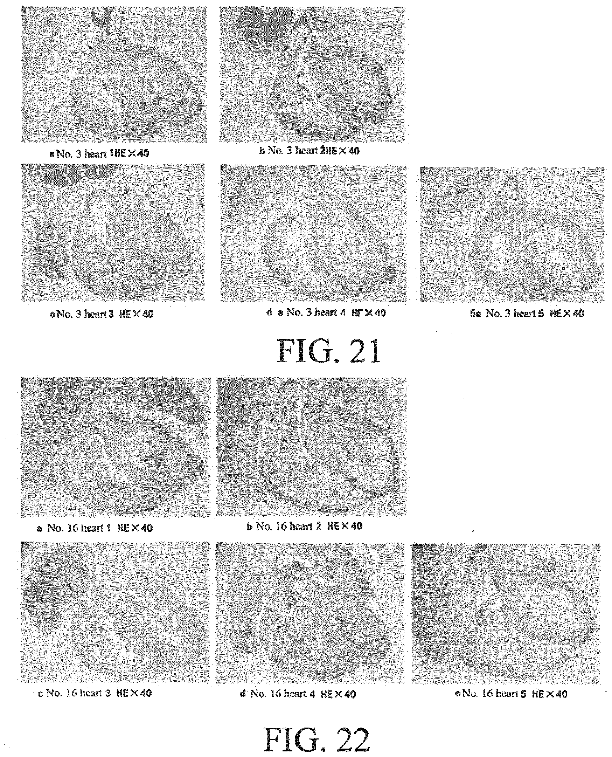

In the folic acid group 2 (2.2775mg/kg), the atrium and the ventricle were basically formed by differentiation, the pericardium, the myocardium, the epicardium and the endocardium were intact, and no necrosis and inflammatory cell infiltration were found. Abundant myocardial trabeculae were seen in the left and right atrium, and only a small section of the left atrium was seen in the several individuals (FIG. 21d). Atrial septal defects were seen in the several individuals (FIG. 21d). The left and right ventricles were basically developed and formed, the myocardial trabeculae in the ventricles were abundant, the connected vascular lumen cound be seen and intravascular valves were visible in individuals (FIG. 21b and e). Atrial and ventricular openings and mitral valves were seen in individual sections (FIG. 21d). Some individuals had left ventricular defects (n=2) or only loosely arranged myocardial trabeculae without obvious cavities (FIG. 21b and c). Some individuals also showed too thin right ventricular walls (FIG. 21b and d).

In the folic acid group 3 (0.911 mg/kg), the atrium and the ventricle were basically formed by differentiation, the pericardium, the myocardium, the epicardium and the endocardium were intact, and no necrosis and inflammatory cell infiltration were found. The left and right atria were relatively full, and the myocardial trabeculae in the atrium were abundant. Only a small part of the left atrium was seen in some individual sections (FIG. 22c and e). The left and right ventricles were basically developed, the left ventricular walls of several individuals were thicker and the cavities were narrow (FIG. 22d). The ventricular wall had abundant blood vessels and the ventricle had abundant myocardial trabeculae. Large blood vessel lumen connected to the ventricles and a valve structure in the lumen could be seen in most sections (FIG. 22a, b, d and e). The mitral valves and right atrioventricular openings were seen in individual sections (FIG. 22c). Several individuals also showed too thin right ventricular walls (FIG. 22c and e). Individual atrial or ventricular walls were defective (n=1).

In the folic acid group 4 (0.4555 mg/kg), the myocardial trabeculae in the left and right atria were abundant, and the atrial section was smaller. The left and right ventricular walls were thicker, the cavities were narrow, the myocardial trabeculae were closely arranged, and the ventricular walls had abundant blood vessels. Several individuals had smaller ventricular sections and stained darker. In the folic acid group 5 (0.22775 mg/kg), the myocardial trabeculae in the left and right atria were abundant. The left and right ventricular walls were thicker, the cavities were narrow, the myocardial trabeculae were closely arranged, the ventricular walls had abundant blood vessels, and the left ventricular cavities were invisible in some individuals (n=5). Large blood vessels and intraluminal valves connected to the ventricles were visible in most sections and only invisible in several individuals.

In the folic acid group 6 (0.113875 mg/kg), the atrium and the ventricle were basically formed by differentiation, the pericardium was intact, the pericardium, intact myocardium, epicardium and endocardium were not seen in sections of only several individuals, and no necrosis and inflammatory cell infiltration were found. The left and right atria were relatively full, and the myocardial trabeculae in the atrium were abundant. In some individuals, the atrial walls were defective (n=1) and there was hematocele in the pericardial cavity. Since there were no corresponding pathological changes, it might be related to sampling. Several individuals had smaller heart. Myocardial trabeculae in the left and right ventricles were abundant, and some individuals had thicker left ventricular walls and narrow left ventricular cavities. Several individuals also showed ventricular septal defects (n=3), right ventricular stenosis or too thin right ventricular walls.

Statistics of all the folic acid groups were as follows.

**Table 3 Effects of folic acid on development of heart of fetal rats**

| | Development delay | Atrioventricular defects/septal defects | Sparse myocardial trabeculae | Malformation cases | Malformation rate | Number of fetal rats |
|---|---|---|---|---|---|---|
| Group 1 | **1** | 3 | 0 | **4** | 12.9% | 31 |
| Group 2 | **2** | **2** | 0 | **4** | 12.1% | 33 |
| Group 3 | **2** | **1** | 0 | 3 | 11.5% | 26 |
| Group 4 | 0 | **5** | 0 | **5** | 17.2% | 29 |
| Group 5 | 0 | **5** | 0 | **5** | 15.2% | 33 |
| Group **6** | 1 | **4** | 0 | **4** | 9.5% | 42 |
| Blank control group | 0 | 0 | 0 | 0 | 0% | 43 |

Due to a small sample size, no statistical induction was made. However, from pathological results, the folic acid was likely to cause ventricular defects or septal defects.

### Example 10 Effects of folic acid and 5-methyltetrahydrofolate on development of fetal mice

75 7-week-old female C57BL/6J mice and 25 8-week-old male C57BL/6J mice were provided by Shanghai Lingchang Biotechnology Co., Ltd. with a certificate number of SCXK (Shanghai) 2018-0003. After the animals were purchased, the animals were fed adaptively for about 10 days until the female mice had a weight about 20 g and the male mice had a weight about 25 g, and an experiment began after body maturation. The female mice were randomly divided into 5 groups according to body weight: a solvent control group, a folic acid (151.66 (µg/kg) group, a folic acid (303.32 µg/kg) group, a calcium 5-methyltetrahydrofolate (converted to folic acid, 151.66 µg/kg) group and a calcium 5-methyl tetrahydrofolate (converted to folic acid, 303.32 µg/kg) group. 1.1275 unit mass of the calcium 5-methyltetrahydrofolate equaled to 1 unit mass of the folic acid.

15 mice were in each group and numbered by a toe clipping method. The mice were intragastrically administrated once a day on the second day after grouping. One week after the pre-administration, male and female mice were caged at a ratio of 3:1, a vaginal plug was observed every day from the next day, and a day when the vaginal plug was seen was recorded as day 0.5 of pregnancy. After 14 days of caging, the female mice stopped the administration, the male mice were removed, and the pregnant female mice were fed alone until litter.

Neonatal mice of the day were taken out and weighed. Some neonatal mice were randomly selected from each group and electrocardiogram (NeoNatal Mouse, iWorx, Dover, NH, USA) was performed on the day of birth.

In addition, 4-5 mice were randomly selected from each group and the 6-day-old small animals were subjected to cardiac echocardiography (Vevo 2100 Imaging System, VisualSonics, Toronto, ON, Canada).

After the detection, thoracic and abdominal cavities of the mice were dissected, cardiac morphology and beating were observed under a stereoscopic microscope (SMZ168, Motic, Xiamen, Fujian, China), and photographs and video recordings were taken (Motic Image Plus 3.0, Motic, Xiamen, Fujian, China). The mice were sacrificed and the hearts, the livers, the kidneys and the lungs were harvested. The hearts and the livers of the neonatal mice were selected from each group and fixed with 4% paraformaldehyde, the fixed hearts and livers were embedded in paraffin, the embedded hearts and livers were sliced and the slices were stained with H&E. A half of the other tissues were immersed in RNA later for fixation and the other half were directly immersed in liquid nitrogen and frozen for a subsequent experiment.

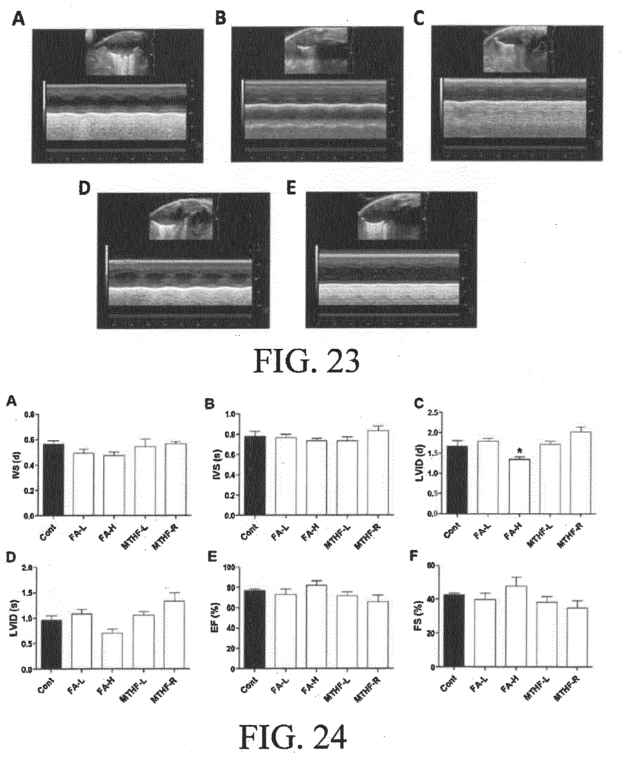

Results were as follows:
Effects of folic acid on cardiac echocardiography of neonatal mice were as follows. The neonatal mice were divided into a control group (A), a low-dose folic acid group (B), a high-dose folic acid group (C), a low-dose methyltetrahydrofolate group (D) and a high-dose methylenetetrahydrofolate group (E). From basic knowledge of kinetics, groups B and C had significantly different dynamic flow phases compared with the control group, while groups D and E were similar to the control group. Echocardiograms of the 6-day-old neonatal mice were seen in FIG. 23.

There was no significant difference in interventricular septum thickness end-diastole (IVS(d)) (mm) and interventricular septum thickness end-systole (IVS(s)) (mm) (A-B); the left ventricular internal diameter end-diastole (LVID(d)) (mm) and the left ventricular internal diameter end-systole (LVID(s)) (mm) in the high-dose folic acid group were significantly lower than those of other groups, and the LVID(d) in the high-dose folic acid group was statistically different from that of other groups (C-D); and there were no significant differences in ejection fraction (EF) and fractional shortening (%) (FS) among groups (E-F). Echocardiogram data of the 6-day-old neonatal mice were seen in FIG. 24.

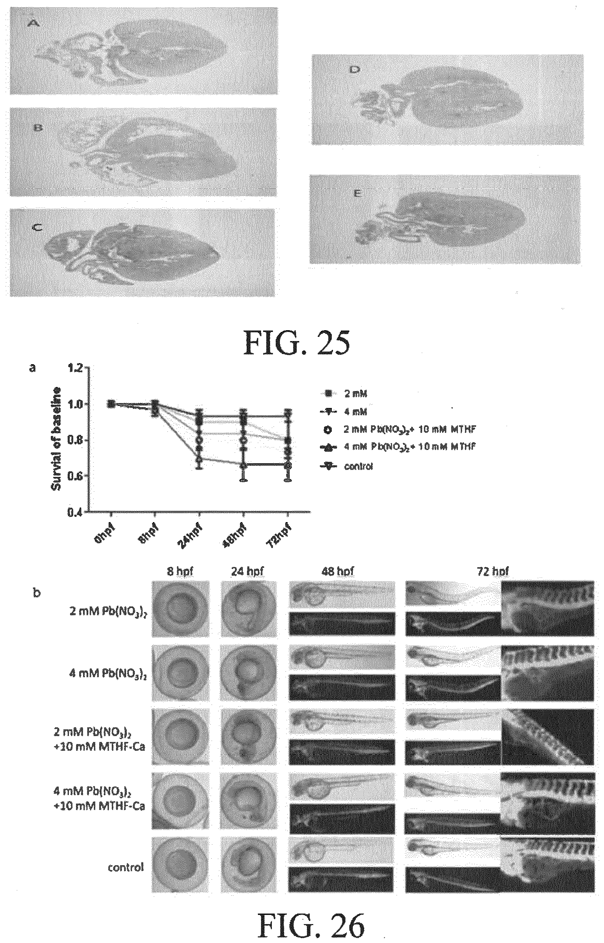

1-day-old neonatal mice were sacrificed, the hearts were taken and fixed with 4% paraformaldehyde, the fixed hearts were embedded in paraffin, the embedded hearts were sliced, the slices were stained with H&E and pictures were taken under a normal microscope. The neonatal mice in the control group, the low-dose methyltetrahydrofolate group and the high-dose methyltetrahydrofolate group had intact myocardium. A small block of a heart wall was defective in a part between the left atrium and ventricle of the neonatal mice in the high- and low-dose folic acid group (B and C) as seen in FIG. 25

**Table 4 Effects of folic acid on development of heart of fetal mice**

| | Development delay | Ventricular defects/septal defects | Sparse myocardial trabeculae | Malformation cases | Number of fetal rats in each group |
|---|---|---|---|---|---|
| Group A | 0 | 0 | 0 | 0 | 90 |
| Group B | 0 | **5** | 0 | **5** | 84 |
| Group C | **2** | 10 | **2** | 10 | 45 |
| Group D | 0 | 0 | 0 | 0 | 68 |
| Group E | 0 | 0 | 0 | 0 | 68 |

Electrodes of the neonatal mice were not inserted subcutaneously like in the adult mice, but were only in contact with the skin, such that measured signals were not very stable and not statistically summarized.

The above results indicated that the calcium 5-methyltetrahydrofolate had no direct ability to cause cardiac development malformations, while the folic acid may cause congenital heart disease.

### Example 11 Calcium 5-methyltetrahydrofolate for preventing cardiac malformations

Transgenic zebrafish (fli-1:EGFP) were from the Model Animal Research Center of Nanjing University. Adult zebrafish aged less than 1 year were used for experiment and fed at a water pH value of 7±0.2, a temperature around 28°C and a ratio of illumination to darkness of 14 h:10 h, and with *Artemia salina* eggs twice a day. Three zebrafish, one female, two males, were placed in a spawning box the night before and eggs were collected the next morning. Fish eggs were placed in an embryo culture medium (prepared with 0.2 g/L of sea salt) and cultured at 28°C.

According to literatures, three different types of substances that have been reported to cause congenital heart disease were selected, namely ethanol, lead nitrate and aristolochic acid. A table was listed below for details.

**Table 4 Rescue agent and drug concentrations in developmental toxicity experiments**

| **Name of drugs** | **Concentration** |
|---|---|
| L-5-MTHF-Ca | 10 mM (maximum solubility) |
| Pb(NO₃)₂ | 2 and 4 mM |
| CH₃CH₂OH | 0.6%, 0.9% and 1.2% |
| Aristolochic acid A | 1, 1.5, 2, 3, 4 and 5 µM |

Zebrafish embryos were cultured in 24-well plates with 10 embryos per well at 2 hpf in a dosing manner, drug solutions of the above concentrations in the table were separately added, termination was conducted at 8 hpf, and the embryos were washed and transferred to an egg solution for continuous culture to 72 hpf. Dead embryos were cleaned up in time. Each experiment was conducted in triplicate.

5-Selected concentrations of methyltetrahydrofolate in the experiment were recorded in the table. A phenotype of malformation was observed, the number of all abnormal zebrafish where the malformation was present was recorded and a malformation rate and a rescue rate of each group were calculated. Morphological defects, a heart rate, cardiac morphology and other indicators were observed and evaluated by an SMZ745T inverted stereomicroscope.

### 1. Effect of 5-methyltetrahydrofolate on preventing cardiac malformations induced by lead nitrate

It was found that 2 and 4 mM Pb(NO₃)₂ reduced a survival rate of embryos, but 10 mM L-5-MTHF-Ca did not rescue an effect of the 2 and 4 mM Pb(NO₃)₂ on a survival rate of the embryos; compared with the control group, phenotypes of the zebrafish embryos at different time points were evaluated; at 8 hpf, the 2 and 4 mM Pb(NO₃)₂ and 2 and 4 mM Pb(NO₃)₂+10 mM L-5-MTHF-Ca did not lead to an obvious embryonic developmental delay; at 24 hpf, the 2 and 4 mM Pb(NO₃)₂ did not significantly affect development and growth of the zebrafish, the zebrafish embryos developed heads and tails, and the tails swung; and at 48 hpf, the 2 and 4 mM Pb(NO₃)₂ did not affect development of intersegmental blood vessels in the zebrafish and did not lead to pericardium edema; and at 72 hpf, the 2 and 4 mM Pb(NO₃)₂ led to a shortened body length of the zebrafish and a large amount of embryonic malformations, mainly manifested as curved bodies and short tails, but there was no significant difference in development of lower intestinal network blood vessels. In contrast, 10 mM L-5-MTHF-Ca could rescue embryonic development malformations induced by 2 and 4 mM Pb(NO₃)₂ (FIG. 26).

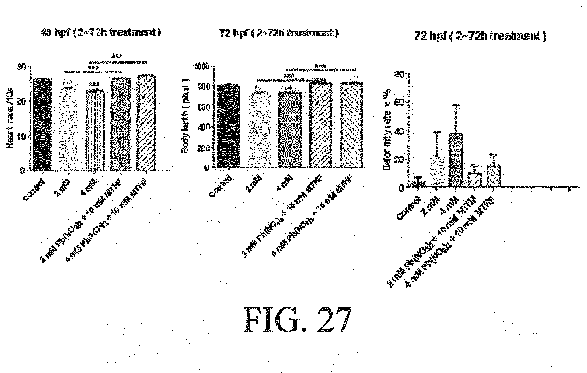

To further quantitatively assess a rescue effect of the 10 mM L-5-MTHF-Ca on Pb(NO₃)₂, since no pericardium edema of embryos showed, a 10 s heart rate of zebrafish embryos was assessed at 48 hpf and a body length of zebrafish among groups was assessed at 72 hpf. It can be seen from FIG. 27, Pb(NO₃)₂ seriously led to bradycardia, a shortened body length and an increased malformation rate of embryos, and showed a significant concentration dependence, while 10 mM L-5-MTHF-Ca could rescue malformed phenotypes of the bradycardia, the shortened body length and abnormally curved bodies.

In conclusion, 10 mM L-5-MTHF-Ca could rescue all development malformations of zebrafish embryos induced by 2 and 4 mM Pb(NO₃)₂.

### 2. Effect of 5-methyltetrahydrofolate on preventing cardiac malformations induced by ethanol

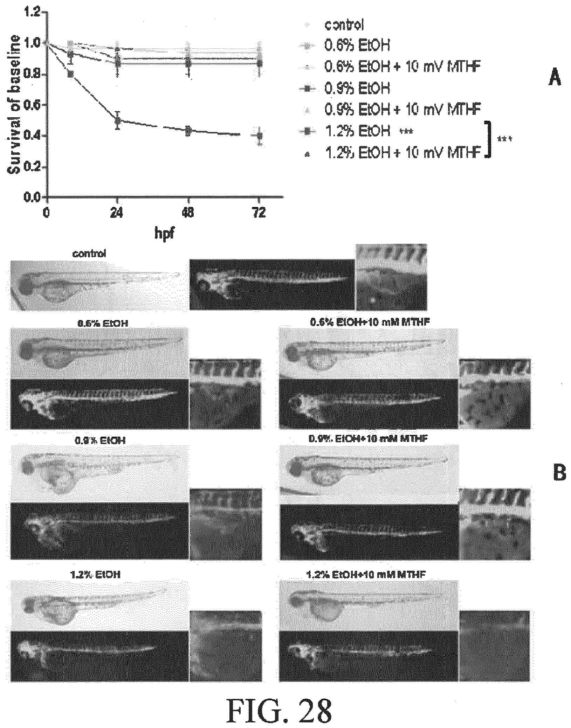

It was found that a survival rate of a 1.2% EtOH group was significantly different from that of the control group, but a 1.2% EtOH+10 mM L-5-MTHF-Ca group could rescue the survival rate and had no difference in the survival rate with the control group (FIG. 28A). At 72 hpf, zebrafish in 0.6% and 0.9% EtOH groups had a similar morphology to that of the control group, 1.2% EtOH led to developmental disorders of lower intestinal network blood vessels of the zebrafish, while 1.2% EtOH+10 mM L-5-MTHF-Ca failed to ameliorate the defect (FIG. 28B).

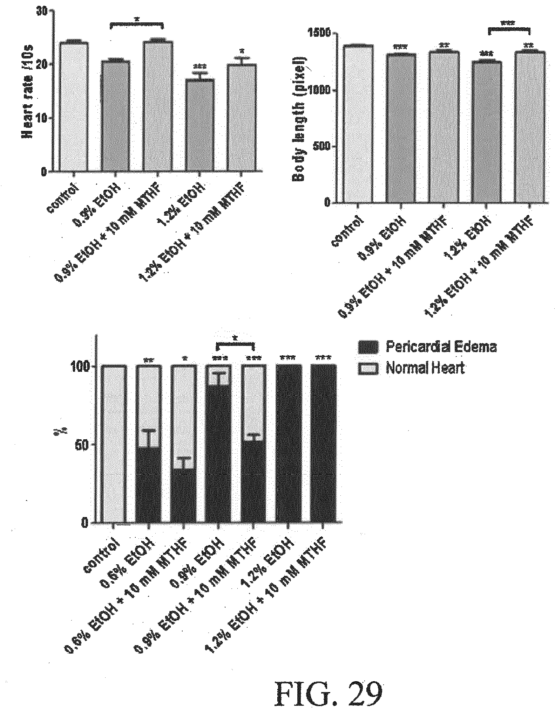

At 48 hpf, 0.9% EtOH and 1.2% EtOH led to a malformed phenotype of bradycardia in zebrafish embryos and had no effect on pigment growth and development of intersegmental blood vessels; and 0.9% and 1.2% EtOH+10 mM L-5-MTHF-Ca could relieve bradycardia. At 48 hpf, EtOH of various concentrations led to a malformed phenotype of pericardium edema in zebrafish embryos and 0.6% EtOH+10 mM L-5-MTHF-Ca could reduce a rate of pericardium edema; and 1.2% EtOH+10 mM L-5-MTHF-Ca had no effect on relieving pericardium edema (FIG. 29).

In conclusion, the ethanol led to early embryonic developmental delay, pericardium edema, bradycardia, a shortened body length, and developmental disorders of lower intestinal network blood vessels in zebrafish embryos, while had no effects on the pigment growth and development of intersegmental blood vessels. The 10 mM L-5-MTHF-Ca could relieve pericardium edema at a low concentration (0.6%) of EtOH, relieve a shortened body length and improve a survival rate of embryos at a high concentration (1.2%) of EtOH, and obviously relieve bradycardia and have a certain preventive effect on malformations at middle and high concentrations (0.9% and 1.2%) EtOH.

### 3. Preventive effect of 5-methyltetrahydrofolate on malformation caused by aristolochic acid A

1-5 µM aristolochic acid A were selected for modeling and 10 mM L-5-MTHF-Ca was used for a rescue experiment. It was found that 2-5 µM aristolochic acid A would seriously reduce a survival rate of embryos, while 10 mM L-5-MTHF-Ca could rescue an effect of the aristolochic acid A on the survival rate of the embryos (FIG. 30), but had no significant rescue effect on a high-concentration aristolochic acid group A (5 µM).

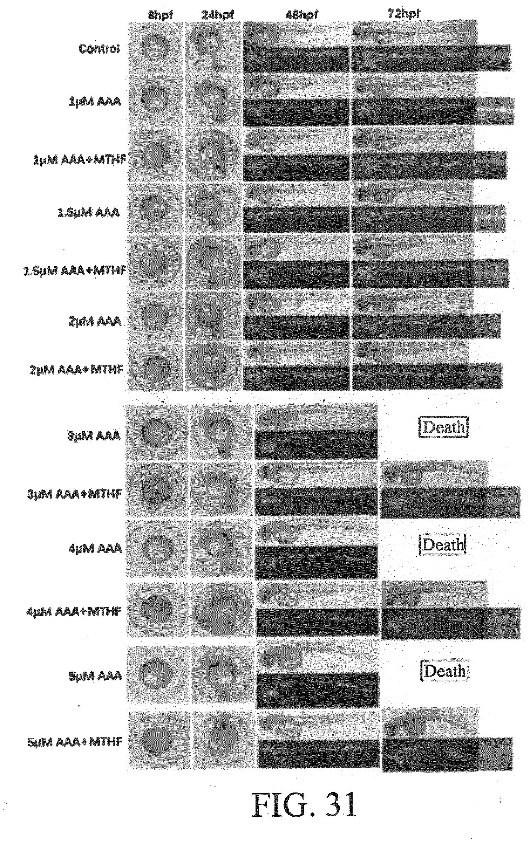

Compared with the control group, phenotypes of zebrafish embryos at different time points were evaluated (FIG. 31). At 8 hpf and 24 hpf, 1-5 µM aristolochic acid A and 1-5 µM aristolochic acid A+10 mM L-5-MTHF-Ca did not lead to obvious embryonic development delay, malformation and death; at 48 hpf, with an increase of aristolochic acid A concentration, the embryos in the aristolochic acid A treatment groups gradually showed pericardium edema, a slowed heart rate, curved bodies, a disappeared systemic blood flow, hematocele, yolk sac opacity, etc., which were aggravated with the increase of the aristolochic acid A concentration; dysplasia of intersegmental blood vessels showed in the high concentration group; while the 10 mM L-5-MTHF-Ca could rescue malformations such as pericardium edema, bradycardia and dysplasia of intersegmental blood vessels caused by the aristolochic acid A; and at 72 hpf, the zebrafish in the aristolochic acid A-treated group showed a complete deficiency of development of the lower intestinal network blood vessels of the zebrafish, the body length was also shortened, and the 10 mM L-5-MTHF-Ca could rescue the shortened body length and the deficiency of the development of the lower intestinal network blood vessels of the zebrafish caused by the aristolochic acid A to a certain extent.

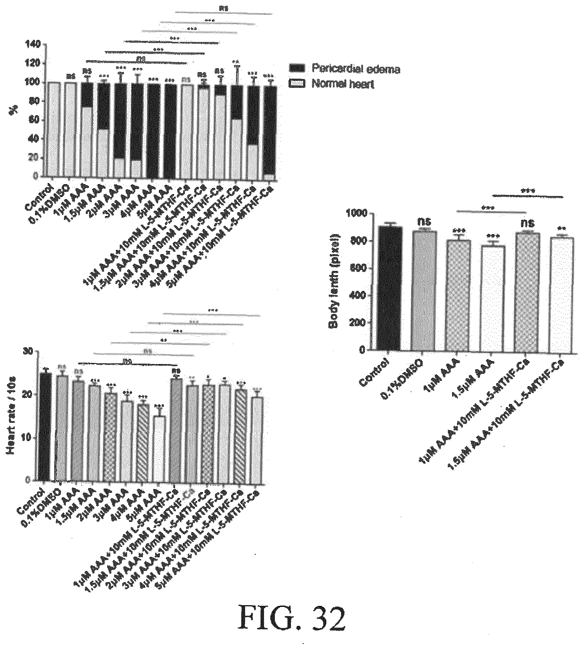

To further quantitatively assess a rescue effect of the10 mM L-5-MTHF-Ca on the aristolochic acid A, pericardium edema and a 10 s heart rate of zebrafish embryos were assessed at 48 hpf, and a body length of zebrafish among groups was assessed at 72 hpf. It can be seen from FIG. 32, 1-5 µM aristolochic acid A seriously led to pericardium edema, bradycardia, and a shortened body length in zebrafish, while 10 mM L-5-MTHF-Ca could rescue malformed phenotypes of the pericardium edema, the bradycardia and the shortened body length of the zebrafish to a certain extent.

In conclusion, the 10 mM L-5-MTHF-Ca could rescue development malformations of zebrafish embryos and death induced by the 1-5 µM aristolochic acid A to a certain extent.

## Claims

1. Use of 5-methyltetrahydrofolate in preparing a medicine or health-care food for preventing neonatal congenital heart disease in peri-pregnancy and/or pregnant women.

2. The use according to claim 1, wherein the congenital heart disease is selected from any one of the following subgroup diseases: 1. Congenital malformation of great arteries, comprising any one of patent ductus arteriosus, aortic stenosis, pulmonary artery stenosis, pulmonary atresia or other congenital malformation of great arteries; 2. Congenital septal defects, comprising any one of atrioventricular septal defects (AVSDs), ventricular septal defects (VSDs), atrial septal defects (ASDs), tetralogy of Fallot, aortopulmonary septal defects or other congenital septal defects; and 3. Other congenital heart disease, comprising any one of congenital malformations of cardiac chambers and connections, congenital aortic or mitral valve malformations.

3. The use according to claim 1, wherein the congenital heart disease is caused by heavy metals, alcohol or medicines.

4. The use according to claim 1, wherein the 5-methyltetrahydrofolate comprises 5-methyltetrahydrofolate or a pharmaceutically acceptable salt thereof.

5. The use according to claim 4, wherein the 5-methyltetrahydrofolate is selected from a group consisting of 5-methyl-(6S)tetrahydrofolate, 5-methyl-(6R)tetrahydrofolate, or 5-methyl(6R, S)tetrahydrofolate, i.e. comprising optical isomers of the 5-methyltetrahydrofolate and a mixture of the optical isomers, especially pure optical natural isomers.

6. The use according to claim 4, wherein the pharmaceutically acceptable salt comprises a corresponding acidic salt formed by converting a basic group of the 5-methyltetrahydrofolate and a corresponding basic salt formed by converting an acidic group of the 5-methyltetrahydrofolate; and the pharmaceutically acceptable salt is preferably a hydrochloride, a sulfate, a nitrate, a phosphate, a sodium salt, a potassium salt, a magnesium salt, a calcium salt, an ammonium salt, a substituted ammonium salt, or a salt formed with arginine or lysine.

7. According to the use of any one of claims 1-6, dosage forms of the medicine or the health-care food comprising an injection, a tablet, a capsule, a pill, an oral liquid, a granule or a powder.

8. Use of 5-methyltetrahydrofolate in preparing a medicine or health-care food for preventing miscarriage or stillbirth in peri-pregnancy and/or pregnant women.

9. The use according to claim 8, wherein the miscarriage or stillbirth is caused by fetal heart development malformations.

10. The use according to claim 8, wherein the miscarriage or stillbirth is caused by peri-pregnancy and/or pregnant women in an environment containing formaldehyde.

11. The use according to claim 8, wherein the 5-methyltetrahydrofolate comprises 5-methyltetrahydrofolate or a pharmaceutically acceptable salt thereof.

12. The use according to claim 11, wherein the 5-methyltetrahydrofolate comprises 5-methyl-(6S)tetrahydrofolate, 5-methyl-(6R)tetrahydrofolate, or 5-methyl(6R, S)tetrahydrofolate, i.e. comprising optical isomers of the 5-methyltetrahydrofolate and a mixture of the optical isomers, especially pure optical natural isomers.

13. The use according to claim 11., wherein the pharmaceutically acceptable salt comprises a corresponding acidic salt formed by converting a basic group of the 5-methyltetrahydrofolate and a corresponding basic salt formed by converting an acidic group of the 5-methyltetrahydrofolate; and the pharmaceutically acceptable salt is preferably a hydrochloride, a sulfate, a nitrate, a phosphate, a sodium salt, a potassium salt, a magnesium salt, a calcium salt, an ammonium salt, a substituted ammonium salt, or a salt formed with arginine or lysine.

14. According to the use of any one of claims 8-13, dosage forms of the medicine or the health-care food comprising an injection, a tablet, a capsule, a pill, an oral liquid, a granule or a powder.

15. Use of 5-methyltetrahydrofolate in preparing a medicine or health-care food for preventing fetal heart malformations.

16. The use according to claim 15, wherein the congenital heart disease is caused by heavy metals, alcohol or medicines.

17. The use according to claim 16, wherein the heart malformations are caused by ethanol, lead nitrate and aristolochic acid.

18. The use according to claim 15, wherein the 5-methyltetrahydrofolate comprises 5-methyltetrahydrofolate or a pharmaceutically acceptable salt thereof.

19. The use according to claim 18, wherein the 5-methyltetrahydrofolate is selected from a group consisting of 5-methyl-(6S)tetrahydrofolate, 5-methyl-(6R)tetrahydrofolate, or 5-methyl(6R, S)tetrahydrofolate, i.e. comprising optical isomers of the 5-methyltetrahydrofolate and a mixture of the optical isomers, especially pure optical natural isomers.

20. The use according to claim 15, wherein the pharmaceutically acceptable salt comprises a corresponding acidic salt formed by converting a basic group of the 5-methyltetrahydrofolate and a corresponding basic salt formed by converting an acidic group of the 5-methyltetrahydrofolate; and the pharmaceutically acceptable salt is preferably a hydrochloride, a sulfate, a nitrate, a phosphate, a sodium salt, a potassium salt, a magnesium salt, a calcium salt, an ammonium salt, a substituted ammonium salt, or a salt formed with arginine or lysine.
